(19) Europäisches Patentamt
European Patent Office
Office européen des brevets

(11) EP 1 295 886 A1

(12) EUROPEAN PATENT APPLICATION

(43) Date of publication:
**26.03.2003 Bulletin 2003/13**

(21) Application number: **01250331.4**

(22) Date of filing: **20.09.2001**

(51) Int Cl.[7]: **C07D 493/08**, C07D 493/18, C07F 7/08, C07D 309/26, C07F 9/655, C07D 407/14 // (C07D493/08, 321:00, 311:00), (C07D493/18, 321:00, 311:00, 311:00)

(84) Designated Contracting States:
**AT BE CH CY DE DK ES FI FR GB GR IE IT LI LU MC NL PT SE TR**
Designated Extension States:
**AL LT LV MK RO SI**

(71) Applicant: **Schering Aktiengesellschaft**
**13353 Berlin (DE)**

(72) Inventors:
- **Mulzer, Johann**
  **1090 Wien (DE)**
- **Enev, Valentin, S**
  **1130 Wien (AT)**

# (54) Laulimalide-derivates, their use and process for the production of laulimalide and laulimalide-derivates

(57) Described are laulimalide-derivatives, their use and a process for the production of laulimalide (Fijanolide B; structure see formula I, in which X and $X_1$ has the meaning of oxygene, $R_1$ has the meaning of α-methyl, $Z_1$ has the meaning of a (E)-double bond, $Z_2$ has the meaning of an β-epoxide and $Z_3$ has the meaning of a (Z)-double bond) and its derivatives. The invention further relates to the intermediates used for the production of laulimalide and laulimalide-derivatives.

EP 1 295 886 A1

## Description

The invention relates to laulimalide-derivatives, their use, and process for the production of laulimalide (Fijanolide B; structure see formula I, in which X and $X_1$ has the meaning of oxygene, $R_1$ has the meaning of $\alpha$-methyl, $Z_1$ has the meaning of a (E)-double bond, $Z_2$ has the meaning of an $\beta$-epoxide and $Z_3$ has the meaning of a (Z)-double bond) and its derivatives. The invention further relates to the intermediates used for the production of laulimalide and laulimalide-derivatives.

The interest in a total synthesis of natural products, such as laulimalide has been kindled by the striking success of paclitaxel as a novel drug against previously incurable tumors. However, in view of the manifold problems met during the clinical application of paclitaxel, a great deal of effort has been focused on potential successors with the same mode of microtubule stabilizing antitumor action, however, with better bioavailability and higher activity against multidrug resistant tumor cells. Among recent advances have been epothilone B and derivatives, discodermolide and eleutherobin. Quite recently, it has been discovered that laulimalide, a metabolite from various marine sponges, also shows microtubule stabilization in eukaryotic cells, however, with better bioavailability and high antitumor activity against multidrug resistant cells lines. To date, only one total synthesis of laulimalide has been published along with several approaches to major fragments (Synthesis of Fragments:

(a) Shimizu, A.; Nishiyama, S. *Tetrahedron Lett.* **1997**, *38*, 6011-6014.
(b) Shimizu, A.; Nishiyama, S. *Synlett* **1998**, 1209-1210) (c) Ghosh, A. K.; Mathivanan, P.; Cappiello, J. *Tetrahedron Lett.* **1997**, *38*, 2427-2430. (d) Ghost, A. K.; Wong, Y. *Tetrahedron Lett.* **2000**, *41*, 2319-2322.
(e) Mulzer, J.; Hanbauer, M. *Tetrahedron Lett.* **2000**, *41*, 33-36.
(f) Dorling, E. K.; Öhler, E.; Mulzer, J. *Tetrahedron Lett.* **2000**, 41, 6323-6326.
(g) Dorling, E. K.; Öhler, E.; Mantoulidis, A.; Mulzer, J. *Synlett* **2001**, 1105-1108.
(h) Nadolski, G. T.; Davidson, B. S. *Tetrahedron Lett.* **2001** *42*, 797-800.
(i) Messenger, B. T.; Davidson, B. S. *Tetrahedron Lett.* **2001** *42*, 801-804.
(j) Paterson, I.; Savi, C. D.; Tudge, M. *Org. Lett.* **2001**, *3*, 213-216.

Total synthesis: Ghost, A. K.; Wong, Y. J. *Am. Chem.* Soc. **2000**, *122*, 11027-11028. *Tetrahedron Lett.* **2001**, *42*, 3399-3402).

There is a high demand for further active compounds which can be used for different deseases, and which show a reasonable bioavailability together with antitumor activity. Further, there is a high demand for an affective process for the production of said natural product compounds.

The disadvantages of the known process is the lower yield combined with the huge amount of process steps.

It has now been found that laulimalid derivatives of general formula I

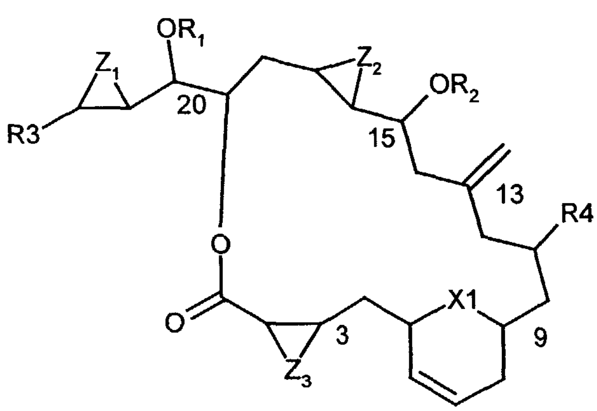

(I),

in which

$R^1$ and $R^2$ independently from each other have the meaning of hydrogen, $\alpha$-alkyl, $\beta$-alkyl, methylmethylether, alkyl

optionally substituted with hydroxy, paramethyloxybenzyl, benzyl, or a protecting group,

$R^3$ has the meaning of a five or six membered, optionally substituted aryl or heteroaryl ring, or a five or six membered optionally substituted and optionally partially saturated cycloalkyl ring, which can be interrupted by oxygen, sulphur or the group $=NR^5$,

$R^4$ has the meaning of α-alkyl, β-alkyl, aryl or trifluoromethyl,

$X_1$ has the meaning of oxygen, sulphur or the group $=NR^5$, in which

$R^5$ has the meaning of hydrogen, alkyl, cycloalkyl or aryl,

$Z_1$ has the meaning of a (Z)- or (E)-double bond, triple bond, or has the meaning of oxygen, sulphur or the group $-CH_2-$, $-CH_2-CH_2-$, $=C(OH)_2$, $=C(halogen)(OH)$, $=C(OH)NR^6$ or $=NR^7$,

$Z_2$ has the meaning of a (Z)- or (E)-double bond, triple bond, or has the meaning of oxygen (α- or β-epoxide), sulphur or the group $-CH_2-$, $-CH_2-CH_2-$, $=C(OH)_2$, $=C(halogene)(OH)$, $=C(OH)NR^6$ or $=NR^7$,

$Z_3$ has the meaning of a (Z) or (E)-double bond,

$R^6$ has the meaning of alkyl, cycloalkyl or aryl, and

$R^7$ has the meaning of hydrogen, alkyl, cycloalkyl or aryl, and the optical isomers and salts thereof, except of those compounds in which $R^3$ stands for the cycloalkyl ring

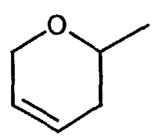

and $X_1$ has the meaning of oxygen, $R^1$ has the meaning of α-methyl, $Z_1$ has the meaning of a (E)-double bond, $Z_2$ has the meaning of β-epoxide if $Z_3$ has the meaning of a (Z)-double bond, overcome the known disadvantages.

The inventive compounds can be used as medicament for the treatment of cancer, such as solide tumors and Leukemia, autoimmune diseases, such as psoriasis, alopezia and multiple sklerose, chemotherapeutically induced alopezia and mukositis, cardiovascular diseases, such as stenosis, arteriosclerosis and restenosis, infectious diseases caused by unicellulare parasites, such as trypanosoma, toxoplasma or plasmodium, or nephrological diseases caused by fungi, such as glomerulonephritis, chronical neurodegenerative diseases, such as Huntington's disease, amyotropical lateral sclerose, Parkinson disease, AIDS dementia and Alzheimer's diseases, acute neurodegenerative disease, such as mia of the brain and neurotraumata, virale infektions, such as wie z. B. Cytomegalus-infectiones, herpes, hepatitis B and C, and HIV diseases.

Thus, the use of the inventive compounds is also claimed matter.

For the use of the compounds of general formula I as medicament, compounds are brought into a pharmaceutical formulation, which beside the active inventive compound further comprises organic or inorganic inert carrier materials, such as water, gelatin, gum Arabic, lactose, starch, magnesia stearate, talc, vegetable oils, polyethylene glycols, etc.

Said formulations can be used for enteral or parenteral applications.

These pharmaceutical formulations can be applied in solid form, such as tablets, pills,suppositories, capsules; or in fluid form, such as solutions, suspensions, or emulsions.

If necessary, these formulations further comprise additives, such as preservatives, stabilizers, or emulgators; and salts for the change of the osmotic pressure or as buffer

For parenteral application special injectable solutions or suspensions, especially liquid solutions of the active compound in polyhydroxy ethoxylated castor-oil are suitable.

As carrier systems it is also possible to use salts, such as gall acid, or plant or animal phospholipids, or a mixture thereof, as well as liposomes or compatiments thereof.

For an oral application, especially tablets, pills or capsules can be used, which comprise talcum, and/ or hydrocarbon carriers or binders, such as lactose, maize or potatoe starch.

The application is also possible as fluid formulation, such as juice, added with sweeteners, or if necessary with one or two artificials.

The dosis of the active ingredients can vary, depending on the way of application, age and weight of the patient, as well as difficilties with respect to hat disease, and other problems.

The daily dosis is 0,5-1000 mg, especially 50-200 mg. The dosis can be applied as single dosis, or separated as two or more daily dosages.

The formulations and appliable forms of the inventive compounds described above, are also claimed matter of the

instant invention.

Further claimed matter are medicaments comprising one or more compounds of general formula I, optionally together with suitable formulations and vehicles, as described above.

It has further been found that the inventive process for the production of laulimalide and laulimalide derivatives overcomes the known disadvantages of the state of the art process.

The retrosynthetic strategy of the process is illustrated in Scheme 1. The key step of the process is the Lewis acid mediated addition of compound II to the acetal moiety of compound III to form the C14 -15-bond.

**Scheme 1**

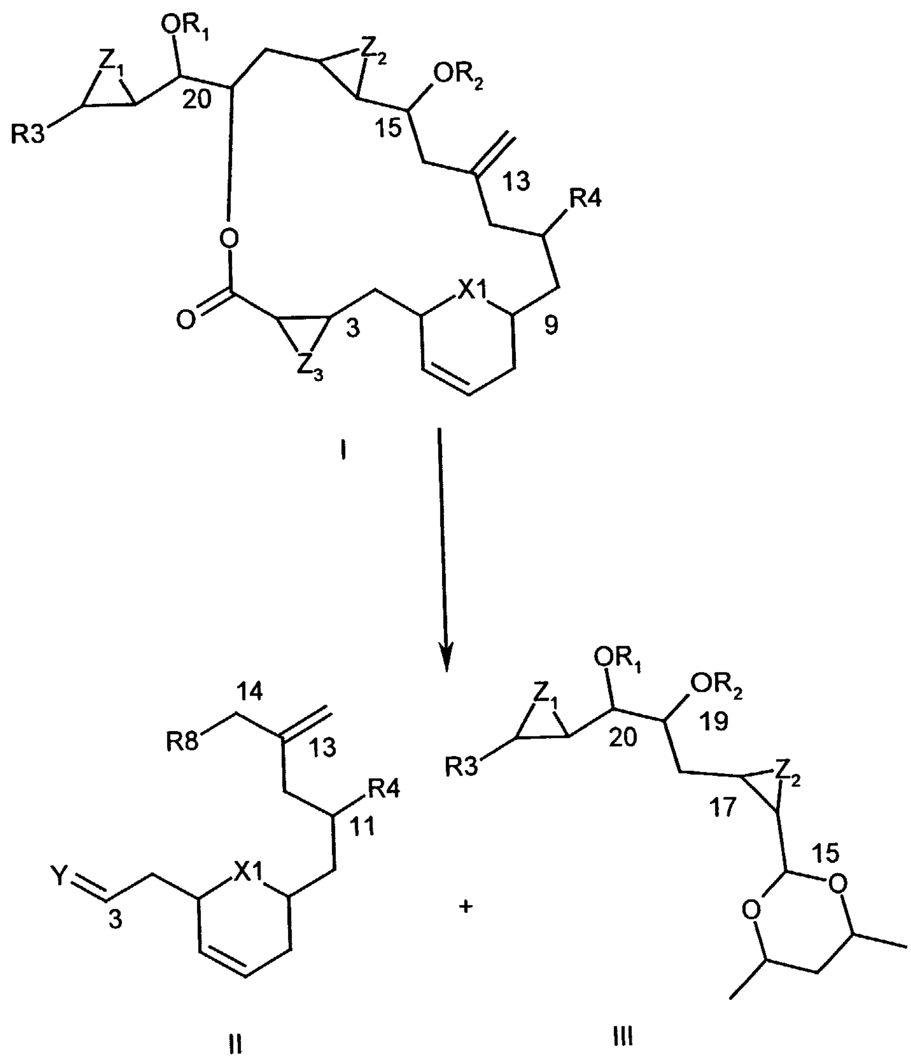

The instant invention therefore concerns a process for the production of laulimalide and laulimalide derivatives of general formula I

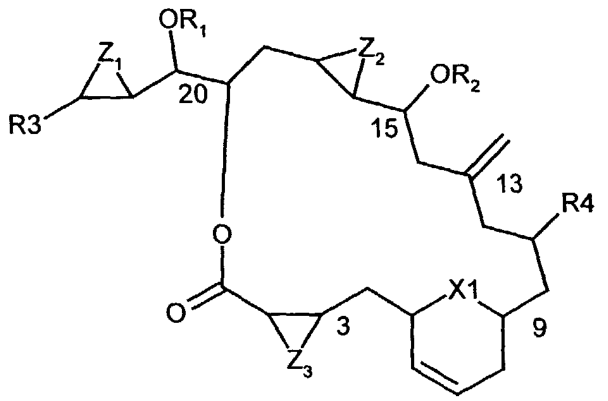

( I ),

in which $R^1$, $R^2$, $R^3$, $R^4$, $X_1$, $Z_1$, $Z_2$, $Z_3$ have the meaning as defined above, characterised in by reacting a compound of general formula II

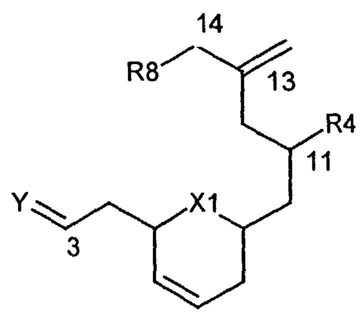

( II ),

in which

| | |
|---|---|
| $R^4$ and $X_1$ | have the meaning defined above under formula I, and |
| $R^8$ | has the meaning of hydrogen, trimethylsilyl, or the group MHal, wherein |
| M | has the meaning of Mg, Li, Ti, Ge or In, and |
| Y | has the meaning of oxygen or the group =CH(OH), (Z) or (E)- CH-COOH, (Z) or (E)- =CH-COOR$^9$ or (Z) or (E)-CHHal, in which |
| $R^9$ | has the meaning of hydrogen, alkyl, cycloalkyl or aryl, |

with a compound of general formula III

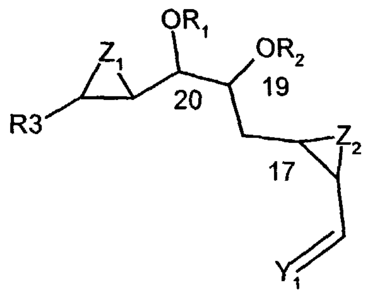

( III ),

in which

| | |
|---|---|
| $R^1$, $R^3$, $Z_1$ und $Z_2$ | have the meaning defined above under formula I, and |
| $R^2$ | has the meaning of hydrogen, methylmethylether, paramethyloxybenzyl, benzyl, or a protecting group, or a group $-COCH_2B$, wherein |
| B | has the meaning of the group $-SiR^{10}$, $-SeR^{11}$, $-Se(O)R^{11}$, $-TeR^{11}$, $-PO(OR^{11})_3$ or $-P(O)(OCH_2CR^{10})_2$, in which |
| $R^{10}$ | has the meaning of alkyl, aryl, alkenyl, or the group $-CF_3$, or $-CH_2OR^{11}$, in which |
| $R^{11}$ | has the meaning of hydrogen, alkyl, cycloalkyl or aryl, and |
| $Y_1$ | has the meaning of oxygen, or an alkyl acetal of the group $-CH(OR^{12})_2$, or a five membered O,O; N,O; O,S; or S,S; cyclic acetal, or six membered O,O; N,O; O,S; or S,S; cyclic acetal, and |
| $R^{12}$ | has the meaning of alkyl, |

to form an intermediate of general formula IV

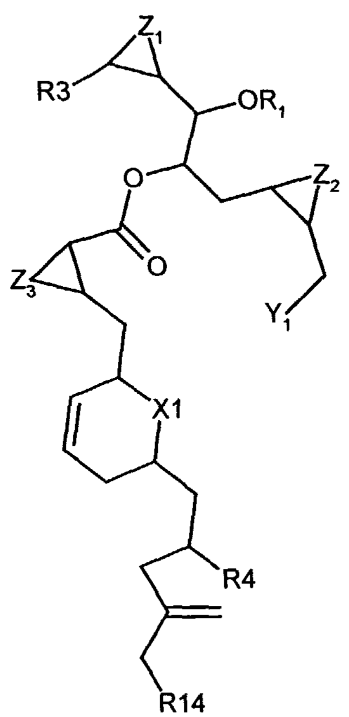

( IV ),

in which

| | |
|---|---|
| $R^1$, $R^3$, $R^4$, $X_1$ and $Z_1$-$Z_3$ | have the meaning defined above under formula I, and |
| $Y_1$ | has the meaning defined above under formula III, and |
| $R^{14}$ | has the meaning of hydrogen, halogen, trimethylsilyl, or the group MHal, wherein |
| M | has the meaning of Mg, Li, Ti, Ge or In, |

which is cyclidized to the compounds of general formula I.

Preferred Laulimalid derivatives of general formula I are those, in which

| | |
|---|---|
| $R^1$ and $R^2$ | independently from each other have the meaning of hydrogen, α-$C_1$-$C_6$-alkyl, β- $C_1$-$C_6$-alkyl, methylmethylether, $C_1$-$C_6$-alkyl optionally substituted with hydroxy, paramethyloxybenzyl, benzyl, or a silyl protecting group, or an acetyl, benzyl, carbamate or carbonate protecting group, |
| $R^3$ | has the meaning of a five or six membered, optionally substituted aryl or heteroaryl ring, or a five or six membered optionally substituted and optionally partially saturated $C_3$-$C_7$-cycloalkyl ring, which can be interrupted by oxygen, sulphur or the group =$NR^5$, |
| $R^4$ | has the meaning of α- $C_1$-$C_6$-alkyl, β- $C_1$-$C_6$-alkyl, aryl or trifluoromethyl, |
| $X_1$ | has the meaning of oxygen, sulphur or the group =$NR^5$, in which |
| $R^5$ | has the meaning of hydrogen, $C_1$-$C_6$-alkyl, $C_3$-$C_7$-cycloalkyl or aryl, |
| $Z_1$ | has the meaning of a (Z)- or (E)-double bond, triple bond, or has the meaning of oxygen, sulphur or the group -$CH_2$-, -$CH_2$-$CH_2$ -, =$C(OH)_2$, =C(halogen)(OH), =C(OH)$NR^6$ or =$NR^7$, |
| $Z_2$ | has the meaning of a (Z)- or (E)-double bond, triple bond, or has the meaning of oxygen (α- or β-epoxide), sulphur or the group -$CH_2$-, -$CH_2$-$CH_2$ -, =$C(OH)_2$, =C(halogene)(OH), =C(OH)$NR^6$ or =$NR^7$, |
| $Z_3$ | has the meaning of a (Z) or (E)-double bond, |
| $R^6$ | has the meaning of $C_1$-$C_6$-alkyl, $C_3$-$C_7$-cycloalkyl or aryl, and |
| $R^7$ | has the meaning of hydrogen, $C_1$-$C_6$-alkyl, $C_3$-$C_7$-cycloalkyl or aryl, |

and the optical isomers and salts thereof, except of those compounds in which $R^3$ stands for the cycloalkyl ring

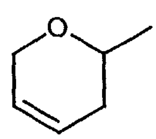

and $X_1$ has the meaning of oxygen, $R^1$ has the meaning of α-methyl, $Z_1$ has the meaning of a (E)-double bond, $Z_2$ has the meaning of β-epoxide if $Z_3$ has the meaning of a (Z)-double bond.

Especially preferred are those laulimalid derivatives of general formula I, according to claims 1 and 2, in which

| | |
|---|---|
| $R^1$ and $R^2$ | independently from each other have the meaning of hydrogen, α-$C_1$-$C_6$-alkyl, β- $C_1$-$C_6$-alkyl, methylmethylether, $C_1$-$C_6$-alkyl optionally substituted with hydroxy, paramethyloxybenzyl, benzyl, or a silyl protecting group, or an acetyl, benzyl, carbamate or carbonate protecting group, |
| $R^3$ | has the meaning of optionally substituted phenyl, biphenyl, naphthyl, thiophene, furan, oxazole, thiazole, imidazole, pyrazole, pyridin, pyrimidine, triazine, quinolin, isoquinolin or benzo derivatives thereof, or a five or six membered optionally substituted and optionally partially saturated $C_3$-$C_7$-cycloalkyl ring, which can be interrupted by oxygen, sulphur or the group =$NR^5$, |
| $R^4$ | has the meaning of α- $C_1$-$C_6$-alkyl, β- $C_1$-$C_6$-alkyl, phenyl, biphenyl, naphthyl, or trifluoromethyl, |
| $X_1$ | has the meaning of oxygen, sulphur or the group =$NR^5$, in which |

| | |
|---|---|
| $R^5$ | has the meaning of hydrogen, $C_1$-$C_6$-alkyl, $C_3$-$C_7$-cycloalkyl, phenyl, biphenyl or naphthyl, |
| $Z_1$ | has the meaning of a (Z)- or (E)-double bond, triple bond, or has the meaning of oxygen, sulphur or the group $-CH_2-$, $-CH_2-CH_2-$, $=C(OH)_2$, =C(halogen)(OH), $=C(OH)NR^6$ or $=NR^7$, |
| $Z_2$ | has the meaning of a (Z)- or (E)-double bond, triple bond, or has the meaning of oxygen (α- or β-epoxide), sulphur or the group $-CH_2-$, $-CH_2-CH_2-$, $=C(OH)_2$, =C(halogene)(OH), $=C(OH)NR^6$ or $=NR^7$ |
| $Z_3$ | has the meaning of a (Z) or (E)-double bond |
| $R^6$ | has the meaning of $C_1$-$C_6$-alkyl, $C_3$-$C_7$-cycloalkyl, phenyl, biphenyl or naphthyl, and |
| $R^7$ | has the meaning of hydrogen, $C_1$-$C_6$-alkyl, $C_3$-$C_7$-cycloalkyl, phenyl, biphenyl or naphthyl, |

and the optical isomers and salts thereof, except of those compounds in which $R^3$ stands for the cycloalkyl ring

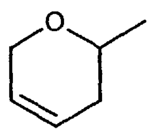

$X_1$ has the meaning of oxygen, $R^1$ has the meaning of α-methyl, $Z_1$ has the meaning of a (E)-double bond, $Z_2$ has the meaning of β-epoxide if $Z_3$ has the meaning of a (Z)-double bond.

Of special interest ara those laulimalid derivatives of general formula I, in which

| | |
|---|---|
| $R^1$ and $R^2$ | independently from each other have the meaning of hydrogen, α-$C_1$-$C_6$-alkyl, β- $C_1$-$C_6$-alkyl, methylmethylether, $C_1$-$C_6$-alkyl optionally substituted with hydroxy, paramethyloxybenzyl, benzyl, or a silyl protecting group, or an acetyl, benzyl, carbamate or carbonate protecting group, |
| $R^3$ | has the meaning of optionally substituted phenyl, biphenyl, naphthyl, thiophene, furan, oxazole, thiazole, imidazole, pyrazole, pyridin, pyrimidine, triazine, quinolin, isoquinolin or benzo derivatives thereof, or a five or six membered optionally substituted and optionally partially saturated $C_3$-$C_7$-cycloalkyl ring, which can be interrupted by oxygen, sulphur or the group $=NR^5$, |
| $R^4$ | has the meaning of α- $C_1$-$C_6$-alkyl, β- $C_1$-$C_6$-alkyl, phenyl, biphenyl, naphthyl, or trifluoromethyl, |
| $X_1$ | has the meaning of oxygen, |
| $Z_1$ | has the meaning of a (Z)- or (E)-double bond, or has the meaning of oxygen, |
| $Z_2$ | has the meaning of a (Z)- or (E)-double bond, or has the meaning of oxygen (α- or β-epoxide) and |
| $Z_3$ | has the meaning of a (Z) or (E)-double bond, |

and the optical isomers and salts thereof, except of those compounds in which $R^3$ stands for the cycloalkyl ring

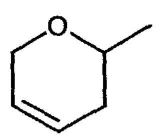

$X_1$ has the meaning of oxygen, $R^1$ has the meaning of α-methyl, $Z_1$ has the meaning of a (E)-double bond, $Z_2$ has the meaning of β-epoxide if $Z_3$ has the meaning of a (Z)-double bond.

Of further special interest are those laulimalid derivatives of general formula I, in which

| | |
|---|---|
| $R^1$ and $R^2$ | independently from each other have the meaning of hydrogen, α-$C_1$-$C_6$-alkyl, β- $C_1$-$C_6$-alkyl, methylmethylether, $C_1$-$C_6$-alkyl optionally substituted with hydroxy, paramethyloxybenzyl, benzyl, or a silyl protecting group, or an acetyl, benzyl, carbamate or carbonate protecting group, |
| $R^3$ | has the meaning of optionally substituted phenyl, 1,3-thiazoles or 2- and 3-pyridyl, or a six membered optionally substituted and optionally partially saturated cyclohexyl ring, which can be interrupted by oxygen, |
| $R^4$ | has the meaning of α- $C_1$-$C_6$-alkyl, β- $C_1$-$C_6$-alkyl, phenyl, biphenyl, naphthyl, or trifluoromethyl, |
| $X_1$ | has the meaning of oxygen, |
| $Z_1$ | has the meaning of a (Z)- or (E)-double bond, or has the meaning of oxygen, |
| $Z_2$ | has the meaning of a (Z)- or (E)-double bond, or has the meaning of oxygen (α- or β-epoxide) and |

$Z_3$ has the meaning of a (Z) or (E)-double bond,

and the optical isomers and salts thereof, except of those compounds in which $R^3$ stands for the cycloalkyl ring

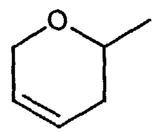

$X_1$ has the meaning of oxygen, $R^1$ has the meaning of α-methyl, $Z_1$ has the meaning of a (E)-double bond, $Z_2$ has the meaning of β-epoxide if $Z_3$ has the meaning of a (Z)-double bond.

Of most interest are those laulimalid derivatives of general formula I, in which

$R^1$ and $R^2$ independently from each other have the meaning of hydrogen, α-$C_1$-$C_6$-alkyl, β- $C_1$-$C_6$-alkyl, methyl-methylether, $C_1$-$C_6$-alkyl optionally substituted with hydroxy, paramethyloxybenzyl, benzyl, or a silyl protecting group, or an acetyl, benzyl, carbamate or carbonate protecting group,

$R^3$ has the meaning of optionally substituted phenyl, 1,3-thiazoles, 2- ai 3-pyridyl or the group

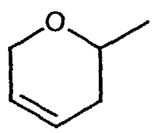

which is substituted with methyl,

$R^4$ has the meaning of α- $C_1$-$C_6$-alkyl, β- $C_1$-$C_6$-alkyl, phenyl, biphenyl, naphthyl, or trifluoromethyl,

$X_1$ has the meaning of oxygen,

$Z_1$ has the meaning of a (Z)- or (E)-double bond,

$Z_2$ has the meaning of a (Z)- or (E)-double bond, and

$Z_3$ has the meaning of a (Z) or (E)-double bond,

and the optical isomers and salts thereof.

Preferred protecting groups are silyl protecting groups, such as trimethylsilyl, triethylsilylyl, tributylsilyl, tributyl-dipropyl-silyl, triisopropylsilyl, t-butyldiphenylsilyl or t-butyldimethylsilyl, or other protecting groups, such as for example acetyl, benzyl, carbamate or carbonate.

Halogene (Hal) has the meaning of chloro, bromo or iodine.

Alkyl stands for a substituted or unsubstituted, linear or branched alkyl chain, such as methyl, ethyl, propyl, iso-propyl, butyl, iso-butyl, sec. butyl, pentyl, iso-pentyl, hexyl, heptyl, octyl, nonyl, decyl, undecyl, dodecyl.

Cycloalkyl stands for a monocyclic alkyl ring, auch as cyclopropyl, cyclobutyl, cyclopentyl, cyclohexyl, cycloheptyl, cyclooctyl, cyclononyl or cyclodecyl; and for a bicyclic ring or tricyclic ring, such as adamantanyl, which each can partially be saturated.

Alkenyl stands for a substituted or unsubstituted, linear or branched alkenyl chain, having 2 - 6, preferred 2 - 4 carbon atoms, such as vinyl, propen-1-yl, propen-2-yl, but-1-en-1-yl, but-1-en-2-yl, but-2-en-1-yl, but-2-en-2-yl, 2-methyl-prop-2-en-1-yl, 2-methyl-prop-1-en-1-yl, but-1-en-3-yl, but-3-en-1-yl, allyl.

Aryl has the meaning of a ring, consisting of 5 - 12 carbon atoms, such as naphthyl, biphenyl, and preferred a phenyl ring.

Heteroaryl has the meaning of a ring, consisting of 4 - 12 carbon atoms, which can be condensed with benzene. For example, a five membered ring has the meaning of thiophene, furan, oxazole, thiazole, imidazole, pyrazole or benzo derivatives thereof; and a six membered ring has the meaning of pyridin, pyrimidine, triazine, quinolin, isoquinolin or benzo derivatives thereof, and preferred thiazole and pyridin, especially 1,3-thiazole and 2- and 3-pyridyl.

Cyclic acetal stands for a ring, such as

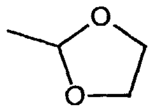
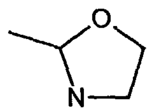
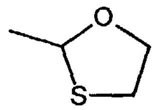
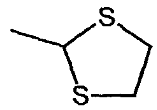

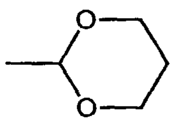
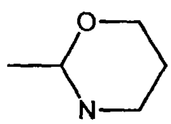
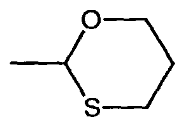
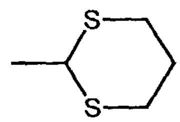

which can optionally be substituted with $C_1$-$C_{10}$-alkyl.

A further matter of the invention are the valuable intermediates which can preferrably be used for the production of laulimalide and ist derivatives.
Therefore, inventive matter are also the intermediates of general formula II

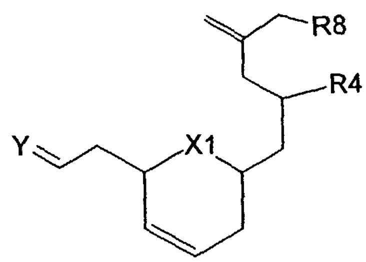

(II),

in which

| | |
|---|---|
| $R^4$ and $X_1$ | have the meaning defined above under formula I, and |
| $R^8$ | has the meaning of hydrogen, trimethylsilyl, or the group MHal, wherein |
| M | has the meaning of Mg, Li, Ti, Ge or In, and |
| Y | has the meaning of oxygen or the group =CH(OH), (Z) or (E)- CH-COOH, (Z) or (E)- =CH-COOR$^9$ or (Z) or (E)-CHHal, in which |
| $R^9$ | has the meaning of hydrogen, alkyl, cycloalkyl or aryl, |

the intermediates of general formula III

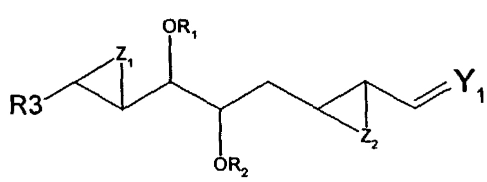

(III),

in which

$R^1$, $R^3$, $Z_1$ und $Z_2$ have the meaning defined above under formula I, and

| | |
|---|---|
| $R^2$ | has the meaning of hydrogene, methylmethylether, paramethyloxybenzyl, benzyl, or a protecting group, or a group $-COCH_2B$, wherein |
| B | has the meaning of the group $-SiR^{10}$, $-SeR^{11}$, $-Se(O)R^{11}$, $-TeR^{11}$, $-PO(OR^{11})_3$ or $-P(O)(OCH_2CR^{10})_2$, in which |
| $R^{10}$ | has the meaning of alkyl, aryl, alkenyl, or the group $-CF_3$, or $-CH_2OR^{11}$, in which |
| $R^{11}$ | has the meaning of hydrogene, alkyl, cycloalkyl or aryl, and |
| $Y_1$ | has the meaning of oxygene, or an alkyl acetal of the group $-CH(OR^{12})_2$, or a five membered O,O; N,O; O,S; or S,S; cyclic acetal, or six membered O,O; N,O; O,S; or S,S; cyclic acetal, and |
| $R^{12}$ | has the meaning of alkyl, |

and the intermediates of general formula IV

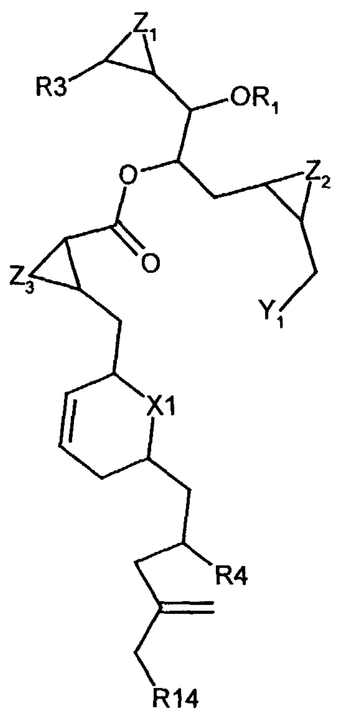

( IV ),

in which

| | |
|---|---|
| $R^1$, $R^3$, $R^4$, $X_1$ and $Z_1$-$Z_3$ | have the meaning defined above under formula I, and |
| $Y_1$ | has the meaning defined above under formula III, and |
| $R^{14}$ | has the meaning of hydrogen, halogen, trimethylsilyl, or the group MHal, wherein |
| M | has the meaning of Mg, Li, Ti, Ge or In, |

for the production of compounds of general formula I.

The intermediates of general formula II,

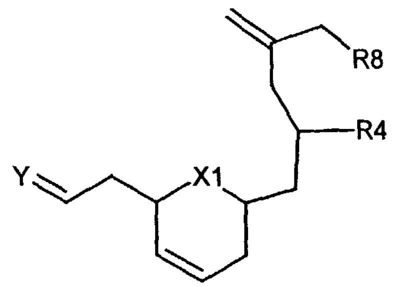

II),

in which $R^4$ and $X_1$ have the meaning as defined under formula I, and $R^8$ has the meaning of hydrogen, trimethylsilyl, or the group MHal, wherein M has the meaning of Mg, Li, Ti, Ge or In, and Y has the meaning of oxygen or the group =CH(OH), (Z) or (E)-CH-COOH, (Z) or (E)- =CH-COOR$^9$ or (Z) or (E)-CHHal, in which $R^9$ has the meaning of hydrogen, alkyl, cycloalkyl or aryl, which are useful for the production of laulimalide and laulimalide derivatives of general formula I, can be produced via a process, which is characterized in by reacting

a) an alcohol compound of general formula II-2)

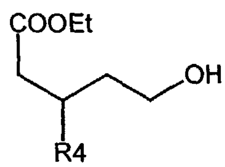

II-2),

in which $R^4$ has the meaning as defined under formula I, in a suitable solvent together with a suitable oxidant to give an aldehyde compound of general formula II-3)

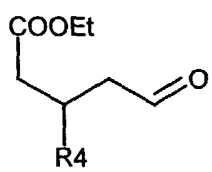

II-3),

in which $R^4$ has the meaning as defined under formula I,

b) reacting aldehyde compound of general formula II-3) in a suitable solvent with a suitable alkylating agent to give a compound of general formula II-4)

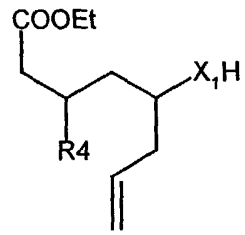

II-4),

in which $R^4$ and $X_1$ have the meaning as defined under formula I,

c) reacting compound II-4) with an amine of general formula II-4a

$$H - NR^iR^{ii} \qquad (II\text{-}4a),$$

in which $R^i$ and $R^{ii}$ independently from each other have the meaning of hydrogen or $C_1$-$C_6$-alkyl, to give a compound of general formula II-5)

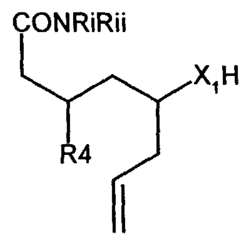

II-5),

in which $R^4$ and $X_1$ have the meaning as defined under formula I and $R^i$ and $R^{ii}$ have the meaning as defined under formula II-4a,

d) reacting compound II-5) in a suitable solvent with a suitable acetal and a suitable hydroxy compound to give a compound of general formula II-6)

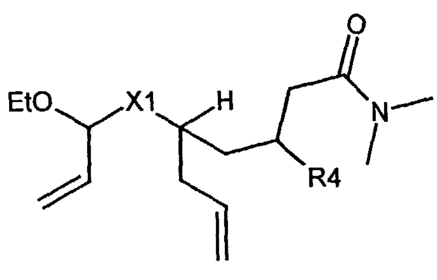

II-6),

in which $R^4$ and $X_1$ have the meaning as defined under formula I,

e) deoxygenating compound II-6) in a suitable solvent with a suitable catalyst, to give a compound of general formula II-7)

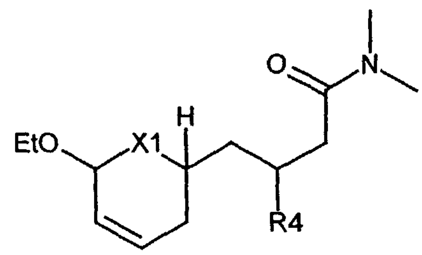

II-7),

in which $R^4$ and $X_1$ have the meaning as defined under formula I,

f) reacting compound II-7) with a suitable vinyl ether and a suitable perchlorate in a suitable solvent, to give a compound of general formula II-8)

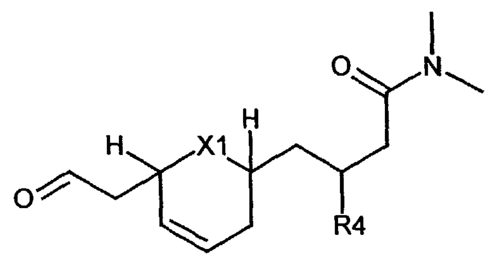

II-8)

in which $R^4$ and $X_1$ have the meaning as defined under formula I,

g) reacting aldehyde compound II-8) with a suitable reductant in a suitable solvent to give an alcohol compound of general formula II-9)

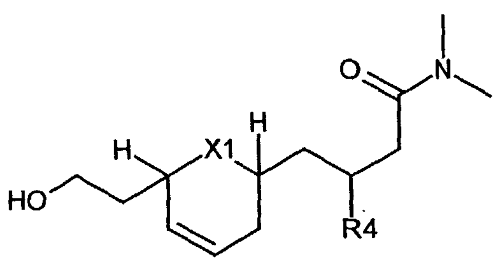

II-9)

in which $R^4$ and $X_1$ have the meaning as defined under formula I,

h) reacting a solution of alcohol compound II-9) in a suitable organic base and with a silyl chloride or a triflak (Si)

of general formula II-9a)

$$R^{21}R^{22}R^{23}\text{-SiCl or } R^{21}R^{22}R^{23}\text{SiOTf} \qquad \text{(II-9a)},$$

in which $R^{21}$, $R^{22}$ and $R^{23}$ independently from each other have the meaning of α- or β-$C_1$-$C_6$-alkyl, to give an amide compound of general formula II-10)

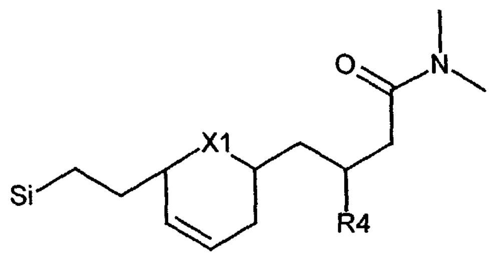

II-10)

in which $R^4$ and $X_1$ have the meaning as defined under formula I and Si has the meaning as defined under formula II-9a,

i) reacting amide compound of general formula II-10) with a metal organic compound and in a suitable solvent to give a ketone compound of general formula II-11)

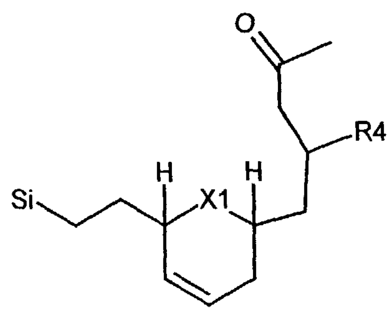

II-11),

in which $R^4$ and $X_1$ have the meaning as defined under formula I and Si has the meaning as defined under formula II-9a,

j) reacting the ketone compound II-11) with a suitable strong amide base in a suitable solvent, and accordingly with a suitable acylating agent in a suitable solvent to give an enoltriflate of general formula II-12)

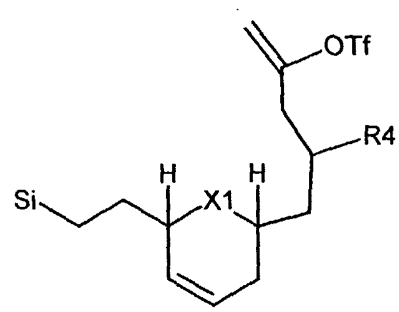

II-12)

in which $R^4$ and $X_1$ have the meaning as defined under formula I and Si has the meaning as defined under formula II-9a,

k) reacting enoltriflate compound II-12) with a suitable Pd catalyst and with alkali chloride in a suitable solvent, and accordingly with $TMSCH_2MgCl$ to give compound II-13)

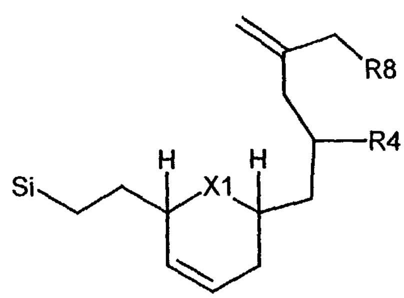

II-13)

in which $R^4$ and $X_1$ have the meaning as defined under formula I, $R^8$ has the meaning as defined under formula II, and Sil has the meaning as defined under formula II-9a,

l) reacting compounds of general formula II-13) with a suitable ionic fluoride in a suitable solvent to give compound II-14)

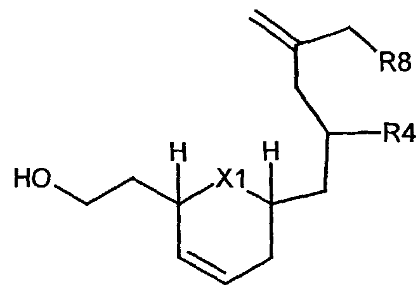

II-14)

in which $R^4$ and $X_1$ have the meaning as defined under formula I, and $R^8$ has the meaning as defined under formula II,

m) reacting compound II-14) with a suitable oxidant in a suitable solvent to give an aldehyde compound of general formula II-15)

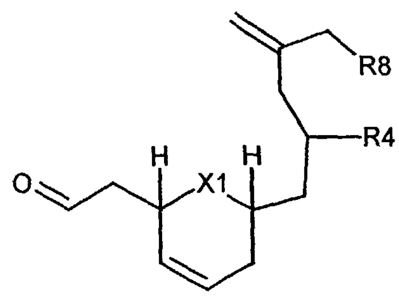

II-15)

in which $R^4$ and $X_1$ have the meaning as defined under formula I, and $R^8$ has the meaning as defined under formula II, and

n) reacting aldehyde compound II-15) with a phosphonyl acetic ester of general formula II-15a)

$$(R^xO)_2P(O)CH_2CO_2R^y \qquad \text{(II-15a)},$$

in which $R^x$ and $R^y$ independently from each other have the meaning of $C_1$-$C_6$-alkyl, halo-$C_1$-$C_6$-alkyl or phenyl, and with 18-crown-6, and a suitable strong amid base, in a suitable solvent to give the intermediate compound of general fomula II).

The intermediates of general formula III,

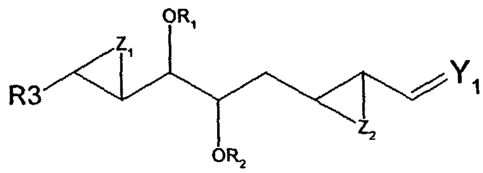

( III ),

in which

| | |
|---|---|
| $R^1$, $R^3$, $Z_1$ and $Z_2$ | have the meaning defined above under formula I, and |
| $R^2$ | has the meaning of hydrogene, methylmethylether, paramethyloxybenzyl, benzyl, or a protecting group, or a group $-COCH_2B$, wherein |
| B | has the meaning of the group $-SiR^{10}$, $-SeR^{11}$, $-Se(O)R^{11}$, $-TeR^{11}$, $-PO(OR^{11})_3$ or $-P(O)(OCH_2CR^{10})_2$, in which |
| $R^{10}$ | has the meaning of alkyl, aryl, alkenyl, or the group $-CF_3$, or $-CH_2OR^{11}$, in which |
| $R^{11}$ | has the meaning of hydrogene, alkyl, cycloalkyl or aryl, and |
| $Y_1$ | has the meaning of oxygene, or an alkyl acetal of the group $-CH(OR^{12})_2$, or a five membered O,O; N,O; O,S; or S,S; cyclic acetal, or six membered O,O; N,O; O,S; or S,S; cyclic acetal, and |
| $R^{12}$ | has the meaning of alkyl, can be produced via a process, |

which is characterized in by reacting

o) an alcohol compound of general Formula III-16)

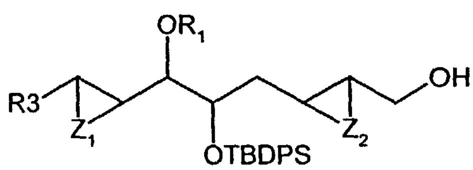

III-16),

in which $R^1$, $R^3$, $Z_1$ and $Z_2$ have the meaning as defined under general formula I, with a suitable oxidant in a suitable solvent to give an aldehyde compound of general formula III-17)

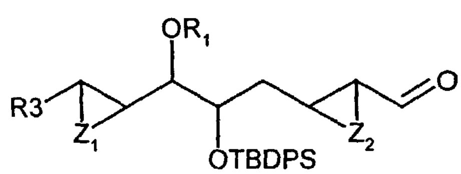

III-17),

in which $R^1$, $R^3$, $Z_1$ and $Z_2$ have the meaning as defined under general formula I,

p) reacting a compound of general formula III-17) with an acetal in a suitable solvent to give a compound of general Formula III-18)

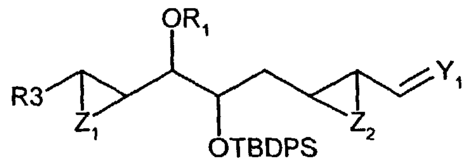

III-18),

in which $R^1$, $R^3$, $Z_1$ and $Z_2$ have the meaning as defined under general formula I, and $Y_1$, has the meaning as defined under formula III,

q) reacting a compound of general formula III-18) with a suitable ionic fluoride in a suitable solvent to an alcohol compound of general formula III-19)

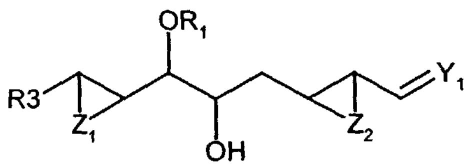

III-19),

in which $R^1$, $R^3$, $Z_1$ and $Z_2$ have the meaning as defined under general formula I, and $Y_1$ has the meaning as defined under formula III, and

r) reacting a compound of general formula III-19) with a suitable tertiary amine in a suitable solvent, and accordingly with a solution of a phosphoro acetyl chloride of general formula III-19a

$$(R^qO)_2P(O)CH_2COCl \qquad (III\text{-}19a),$$

in which $R^q$ has the meaning of $C_1$-$C_6$-alkyl, halo-$C_1$-$C_6$-alkyl or aryl, in a suitable organic base to give a compound of general formula III.

According to the process for the production of the intermediates supra,
a suitable solvent in reaction step a), b), d), e), f), g), i), j), k), l), m), n), o), p), q) and r) is for example ether, tetrahydrofuran, methylene chloride, etc.;
a suitable oxidant in reaction step a), m) and o) is for example 1,1,1-triacetoxy-1,1-dihydro-1,2-benz-iodoxol-3(1H)-one (Dess-Martin periodinane), a combination of dimethylsulfoxide, oxalyl chloride or a tertiary amine (Swern-oxidation), etc.;
a suitable alkylating agent in reaction step b) is for example allyl-diisopinocamphenyl-borane ($Ipc_2Ball$), or alkyl-cyclopentadienyl-[2,2-dimethyl-α,α,α,'α'-tetra-phenyl-1,3-dioxolanyl]-titanium, etc.;
a suitable acetal in reaction step d) and p) is for example acrolein-diethylacetal, etc.;
a suitable hydroxy compound in reaction step d) is for example an alcohol, such as ethanol, etc.;
a suitable vinyl ether in reaction step f) is for example vinyl-tert-butyl-dimethyl-silyl-ether, etc.;
a suitable catalyst in reaction step e) and f) is for example di-chloro-ditriphenyl-phosphino-benzylidene-ruthenium, etc.;
a suitable reductant in reaction step g) is for example sodium borhydride, lithium aluminium hydride, diisobutylaluminium-hydride, lithium borohydride, etc.;
a suitable metal organic compound in reaction step i) is for example methyl lithium, methyl magnesium bromide, etc.;
a suitable alkali chloride in reaction step k) is for example LiCl, NaCl, KCl, RbCl, CsCl, etc.,
a suitable a strong amide base in reaction step j) and n) is for example KHMDS, NaHMDS, LDA, dicyclohexyl-isopropyl-lithium amide, etc.;
a suitable acylating agent in reaction step j) is for example $PhN(OTf)_2$ or $Tf_2O$, tosyl chloride, mesyl chloride, etc.;
a suitable Pd catalyst in reaction step k) is for example $Pd(Ph_3P)_4$, tris-di-benzylideneacetone-dipalladium, etc.;
a suitable ionic fluoride in reaction step l) and q) is for example tetra-n-butylammoniumfluoride (TBAF), hydrogen fluoride in acetonitrile, pyridine, etc.;
a suitable tertiary amine in reaction step r) is for example 4-dimethylamino-pyridine (DMAP), pyridine, collidine, etc.;
a suitable organic base in reaction step h) and r) is for example triethylamine, pyridine, 2,6-ditert-butylpyridine, ethyldiisopropyl amine, etc.;
a suitable perchlorat in reaction step f) is for example lithium perchlorate, etc.

The following process examples describe the production of the inventive intermediate compounds, and further describe the feasability of the inventive process, however, not restricting the process to compounds decribed in these process examples.

### 1.0 Process Examples

### 1.1 In General

Unless otherwise stated, solvents were dried by distillation under argon, from Na (toluene), Na / K ($Et_2O$), potassium (THF), $CaH_2$ ($Et_3N$, DMF), $P_2O_5$ ($CH_2Cl_2$), KOH (pyridine) and Mg (MeOH, EtOH). All other commercially available reagents were used without further purification unless specified otherwise. All reactions were performed in oven-dried glassware under argon. Chromatography refers to flash column chromatography on silica gel 60 (230-400 mesh). Thin layer chromatography (TLC) was performed on Al-backed plates (Merck silica gel 60 $F_{254}$) and visualised by using either a UV lamp, phosphomolybdic acid, sulphuric acidic/anisaldehyde or potassium permanganate solutions. Melting points (mp) are uncorrected. Optical rotations are reported in g/100 ml. Infrared spectra (IR) were measured as evaporated films on single crystal silica plates and reported in wave numbers ($cm^{-1}$) with broad signals denoted by (br). High resolution mass spectra were obtained using electron ionisation (EI), field ionisation (FI) or fast atom bombardment (FAB). $^{1}H$ and $^{13}C$ NMR were recorded on a Bruker AC 250 (250 MHz), AM 400 (400 MHz) or AM 600 (600 MHz) spectrometer. Chemical shifts are reported using the solvent resonance internal standard (chloroform, 7.26 and 77.0 ppm).

### 1.2 Process for the production of the used intermediates

### 1.2.1 Process for the production of intermediate compound II

The production of compound **II**, in which $R^4$ has the meaning of β-methyl, $R^8$ has the meaning of trimethylsilyl, $X_1$ has the meaning of oxygene, and Y has the meaning of group (Z)- =COOMe, was performed according to Scheme 2.

**Scheme 2**

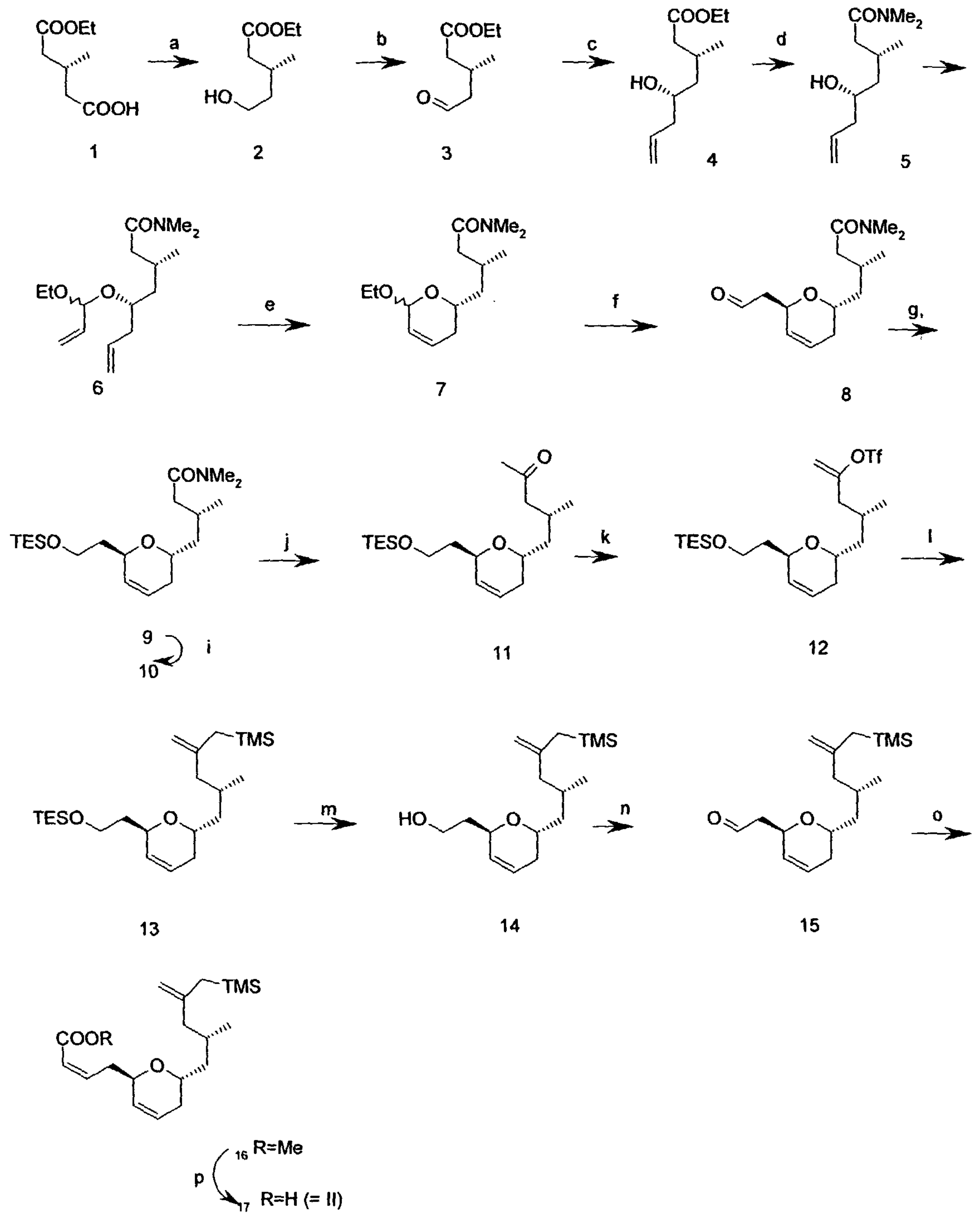


1 2 3 4 5 6 7 8 9 11 12 13 14 15

Reagents and conditions

a) $BH_3.Me_2S$, 98%; b) $Et_2NH$, EtOH, 94%; c) Swern ox. 96%; d)AllylTi TADDOL; 92%, e) acrolein -diethylacetal, TsOH, Tol, 94%; f) 4 mol % Grubs catalyst, $CH_2Cl_2$, 87%; f) TBSO-CH=$CH_2$, $LiClO_4$, 92%; g) $NaBH_4$, MeOH, 0°C, 99%; i) TESCl, Py, Tol, 92%; j) MeLi, $Et_2O$, -75°C->r.t., 90%; k) KHMDS, $C_6H_5N(OTf)_2$, THF, 60%; l) 5 mol% Pd $(PPh_3)_4$, LiCl 5 eqv, $TMSCH_2MgBr$ 5 eqv, $Et_2O$, 96%; m) $K_2CO_3$, MeOH, 0°C, 98%, n) $Py.SO_3$, TEA, DMSO, $CH_2Cl_2$, 90%; o) $MeOOCCH_2P(O)$-$(OCH_2CF_3)_2$, KHMDS, THF, 84%; p) $LiCl.H_2O$, aceton, 0°C, 2 days, 80%.

### 1.2.2 Process for the production of intermediate compounds III and IV

The synthesis of intermediate compound **III** ($R^1$= methylmethylether (MOM), $R^2$= group -$COCH^2B$, B= -P(O)($OCH_2CR^{10})_2$, $R^{10}$= -$CF_3$ (TBDPS), $R^3$ =methyl, X = oxygene, Y= chiral six membered cyclic acetal, Z and $Z_1$ = double bond) and intermediate compound **IV** ($R^1$ = methylmethylether (MOM), $R^3$ = methyl, X, $X_1$ = oxygene, Z and $Z_1$ = (E)-double bonds, $R^2$ = trimethylsilyl (TMS), $Z_3$ = (Z)-double bond, $R^4$ = $\alpha$-methyl) was performed according to Schemes 3 and 4.

## Scheme 3

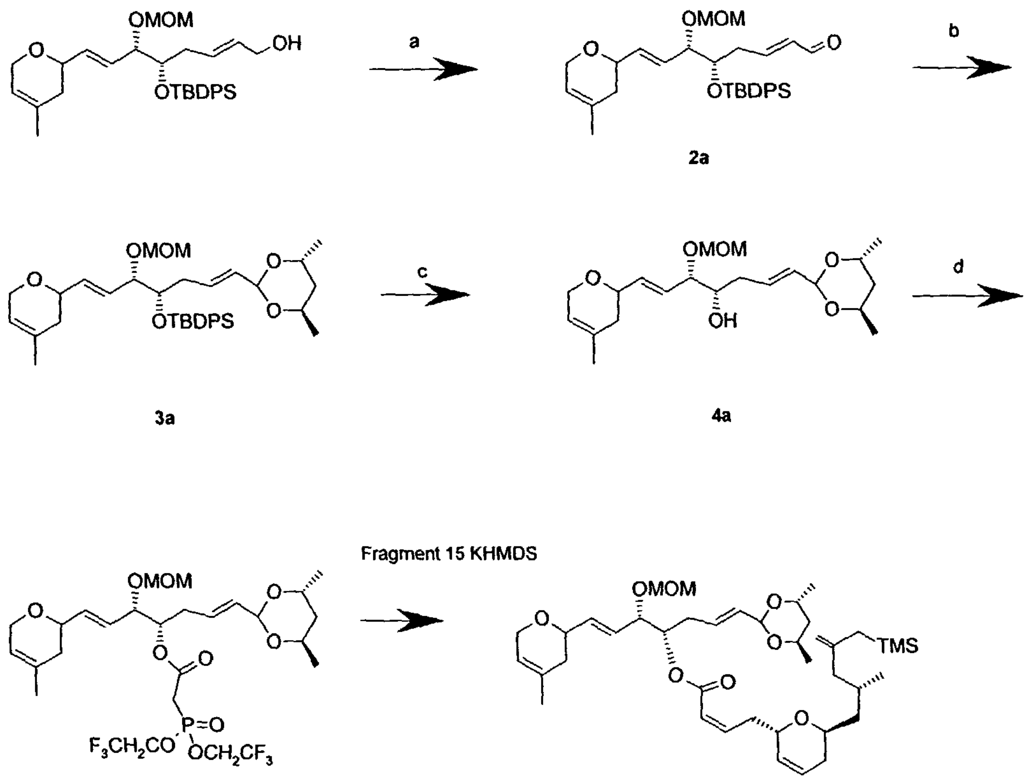


2a

3a

4a

5a (= III)

6a (= IV)

Reagents and conditions: a) Dess-Martin oxidation 96%, b) (R;R)-(-)-2,4 pentan diol/H+, 89%, c) TBAF, THF 96%, d) $(CF_3CH_2O)_2P(O)CH_2COCl$/DMAP/Py, -78°C-r.t, 96%,

**1.2.3 Process for the production of inventive compounds from intermediate of formula IV**

## Scheme 4

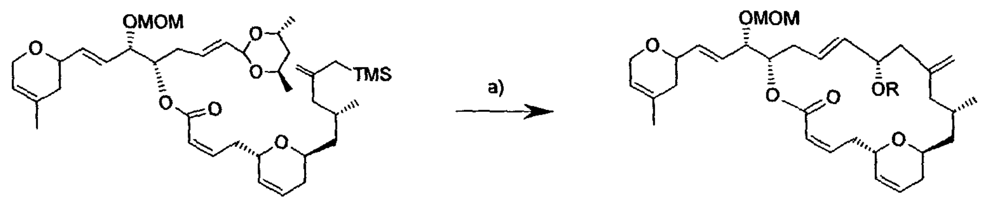


**6a (= IV)**

**7a,** (= I for R = 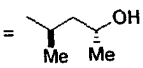 )

Reagents and conditions: a) $EtAlCl_2$, methylene choride, -50-0°C, 85%.

**1.2.4 Process for the selective removal of the O-15- and O-20-protecting groups and for the selective epoxidation of the 16,17-double bond, selective derivatization of intermediates of formula I (Scheme 5)**

## Scheme 5

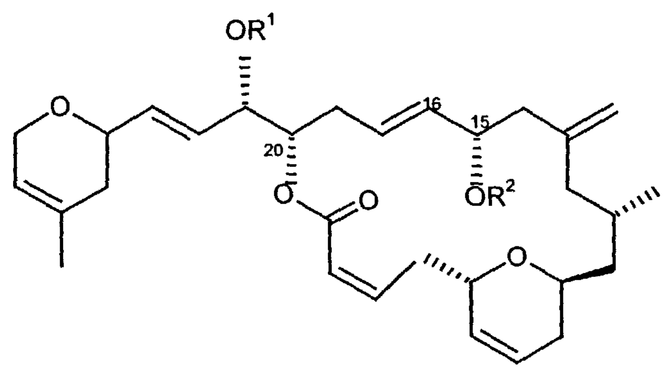

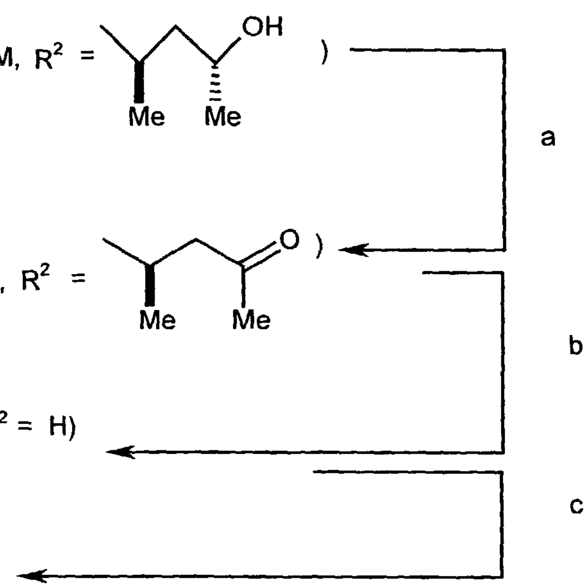


7a(=I for $R^1$ = MOM, $R^2$ = )

7b(=I for $R^1$ =MOM, $R^2$ = )

7c (=I for $R^1$ = MOM, $R^2$ = H)

7d(=I for $R^1$ = $R^2$ = H

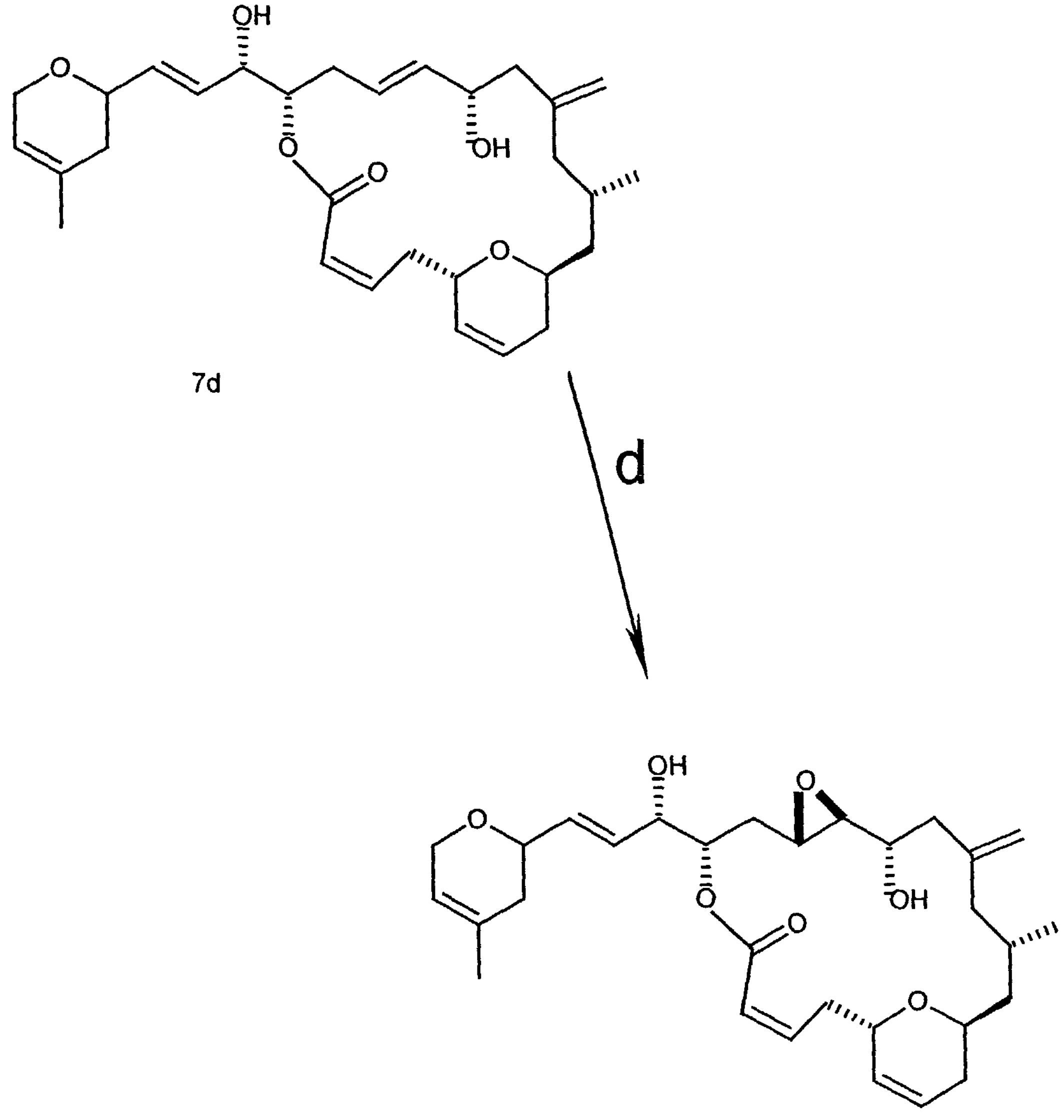


7d

7e (=I, laulimalide)

**Reagents and Conditions:**

a) Dess Martin periodinane, ether, 90%; b) pTsOH, $CH_2Cl_2$, 80%;
c) $Me_2BBr$, ether, -78°C, 96%; d) tBuOOH, $Ti(OiPr)_4$, $CH_2Cl_2$, -20°C, 2h

## Compound **7b**.

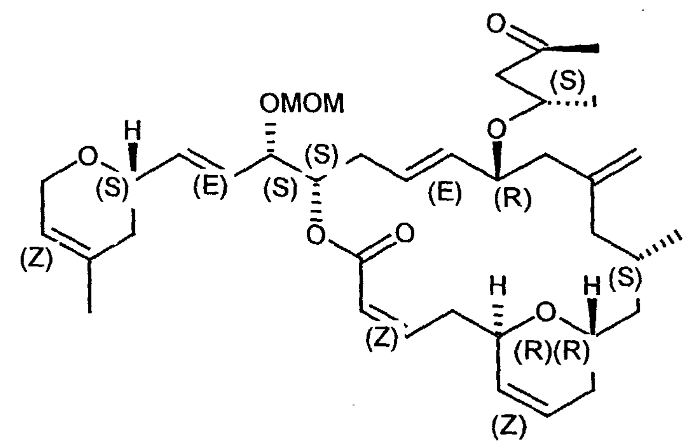

$C_{37}H_{54}O_8$
Exact Mass: 626,3819
Mol.Wt: 626,8199
C, 70,90; H, 8,68; O, 20,42

A solution of alcohol **7a** (0.02g, 0.032mmol) in dry $CH_2Cl_2$ (0.5ml) was added to a slurry of Dess-Martin periodinane (0.02g, 0.06mmol) and $NaHCO_3$ (0.05g) in $CH_2Cl_2$ (2 ml). The mixture was stirred at room temperature for 30 minutes, then subjected directly to $SiO_2$ Flash chromatography (hexane : ethyl acetate - 1:1) to yield **7b** as a colorless oil (0.018g, 90%), $[\alpha]_D^{20}$= -35° (c=0.05 in $CHCl_3$); IR (neat): 2978, 1719, 1682, 1640, 1360, 1166, 1035cm$^{-1}$; $^1$H-NMR (600 MHZ, $CDCl_3$); δin ppm= 6.34 (ddd, *J*=11.5, 10.0, 5.5 Hz), 5.93 (1H, d, *J*=11.6, Hz), 5.85 (2H, m), 5.71 (1H, ddd, *J*=10.2, 4.2, 2.4Hz), 5.62.(1H, m), 5.58 (2H, m), 5.44 (2H, m), 5.09, (1H, dd, *J*=12.9, 6.4 Hz), 4.80 (1H, brs), 4.77 (1H, brs), 4.69 (1H, d, *J*=6.8 Hz), 4.57 (1H, d, *J*=6.8Hz), 4.27 (1H, m), 4.16 (2H, m), 4.16 (2H, m) 4.07 (1H, m), 3.94 (2H, m), 3.83 (1H, ddd *J*=8.3, 8.0, 4.7 Hz), 3.69 (1H, m), 3.40 (3H, s), 2.69. (1H,dd, *J*=15.3, 6.9 Hz), 2.42. (1H, dd, *J*= 15.3, 6.0 Hz), 2.37 (2H, m), 2.26. (1H, dd, *J*= 14.6, 7.9 Hz), 2.16 (3H, s), 2.12 (1H, m), 2.06 (3H, m), 1.90 (2H, m), 1.74 (2H, m), 1.65 (3H, brs), 1.62 (3H, m), 1.16 (1H, m), 1.14 (3H, d, *J*=6.3 Hz), 0.91 (1H, m), 0.87 ( 3H, d, J=6.2 Hz); $^{13}$C-NMR (150 MHz, $CDCl_3$), δ in ppm=208.2 (s), 165.7 (s), 147.7 (d), 144.9 (s), 136.2 (d), 134.7 (d), 131.7 (s), 128.9 (d), 127.9 (d), 126.3 (d), 125.4 (d), 121.8 (d), 120.1 (d), 113.6 (t), 94.4 (t), 77.4 (d), 76.1 (d), 74.3 (d), 73.6 (d), 72.4 (d), 69.0 (d), 67.4 (d), 65.9 (t), 56.0 (d), 51.7 (q), 45.3 (t), 43.2 (t), 42.9 (t), 36.0 (t), 34.5 (t), 33.3 (t), 31.8 (t), 31.7 (d), 28.9 (q), 23.3 (q), 20.6 (q), 19.8 (q); HRMS: found: 626. 8100, $C_{37}H_{54}O_8$ requires 626.8199.

## Compound **7c**

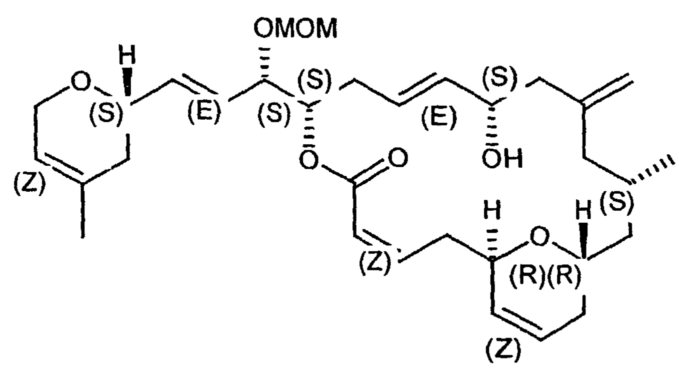

$C_{32}H_{46}O_7$
Exact Mass: 542,3244

Mol Wt: 542,7034
C, 70,82; H, 8,54; O, 20, 64

A solution of **7b** (0.015g, 0.023 mmol) and TsOH (0.002g, 4 eqv) in $CHCl_3$ (5ml) was stirred at 0°C for 24 hours. The mixture was washed with a solution $NaHCO_3$, water followed by brine, dried ($Na_2SO_4$) and concentrated. Flash chromatography of the crude product (hexane: ethyl acetate: 1:1) gave **7c** as a colorless oil (0.010g, 80%); $[\alpha]_D^{20}$= -22° (c=0.05 in $CHCl_3$); IR (neat): 3520, 2990, 1720, 1675, 1640, 1370, 1160, 1022 $cm^{-1}$; $^1$H-NMR ( 600 MHz, $CDCl_3$);
δ in ppm=6.34 (ddd, *J*=11.5, 10.0, 5.3Hz), 5.93 (1H, dt, *J*=11.6, 1.2 Hz), 5.85 (2H, m), 5.72 (1H, ddd, *J*=10.2, 4.1, 1.9 Hz), 5.61.(3H, m), 5.41 (1H, brs), 5.11 (1H, ddd *J*=9.7, 6.0, 3.5 Hz), 4.83 (2H, brs), 4.68 (1H, d, *J*=6.9 Hz), 4.55 (1H, d, J=6.9 Hz), 4.16 (5H, m), 4.06 (1H, m), 3.92 (1H, m), 3.58 (1H,m), 3.35 (3H, s), 2.4-2.0 (10H), 1.9-1.7 (5H, m), 1.69 (3H, brs), 1.64 (2H, m), 1.14 (1H, m), 0.89 (1H, m), 0.86 (3H, d, *J*=6.5 Hz); $^{13}$C-NMR (150 MHz, $CDCl_3$), in ppm=165.3 (s), 146.8 (d), 144.8 (s), 136.0 (d), 135.3 (d), 131.2 (s), 128.3 (d), 126.6 (d), 125.8 (d), 124.7 (d), 121.8 (d), 119.7 (d), 114.3 (t), 93.8 (t), 73.8 (d), 73.1 (d), 71.2 (d), 69.7 (d), 67.4 (d), 65.5 (t), 55.6 (q), 44.7 (t), 43.3 (t), 42.2 (t), 35.7 (t), 34.3 (t), 33.4 (t), 30.9 (t), 29.7 (t), 28.2 (q), 22.2 (q), 19.8(q); HRMS: found: 542.3200, $C_{32}H_{46}O_7$ requires 542.3244.

## Compound **7d**

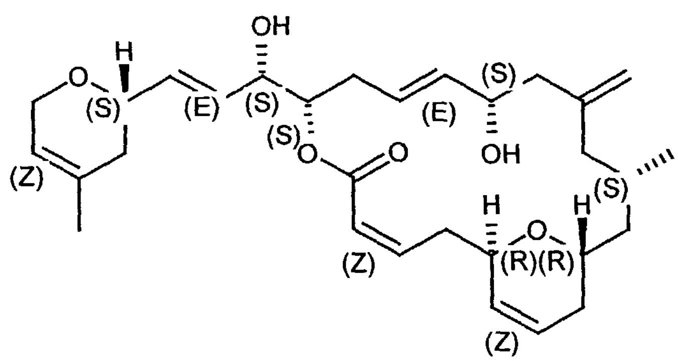

$C_{30}H_{42}O_6$
Exact Mass: 498,2981
Mol Wt: 498,6509
C, 72,26;H 8,49; O, 19,25

To a solution of **7c** (0.01g, 0.18 mmol) in $CH_2Cl_2$ (5ml) was added at -78°C a solution of $Me_2BBr$ (0.3ml, 0.3M in $CH_2Cl_2$) and the mixture was kept at same temperature for 30 min. The reaction was quenched with a mixture of THF and saturated solution of $NaHCO_3$ (1ml). The organic phase was washed with water followed by brine, dried ($Na_2SO_4$) and concentrated. Flash chromatography of the crude product ( hexane: ethyl acetate- 1:1) gave **7d** as a colorless oil (0.09g, 96%); $[\alpha]_D^{20}$=-26°C (c=0.01 in $CHCl_3$); IR (neat): 3422, 2924, 1718, 1654, 1320, 1130, $cm^{-1}$; $^1$H-NMR (600 MHz, $CDCl_3$); δ in ppm=6.32 (1H; ddd, *J*=11.5, 10.0, 5.3 Hz), 5.94 (1H, d, *J*= 11.5Hz), 5.91 (1H, ddd, *J*= 15.6, 5.6, 1.2, Hz), 5.86 (1H, m), 5.76 (2H, m) 5.65 (1H,m), 5.41 (1H, brs), 5.02 (1H, dd, *J*=13.3, 5.8), 4.88 (2H, brs), 4.2-4.1 (5H, m), 4.07 (1H, m), 3.89 (1H, m), 3.55 (1H,m), 2.4-2.1 (6H, m), 2.07 (1H, m), 1.93 (1H, d, *J*=16.9 Hz), 1.85-1.70 (4H, m), 0.87 (3H, d, *J*= 6.3Hz); $^{13}$C-NMR (150 MHz, $CDCl_3$): δ=165.5 (s), 147.8 (d), 144.8 (s), 135.9 (d), 134.0 (d), 131.2 (s), 128.6 (d), 128.3 (d), 126.6 (d), 125.4 (d), 121.6 (d), 119.7 (d), 114.5 (t), 74.0 (d), 73.2 (d), 73.1 (d), 71.2 (d), 69.8 (d), 67.6 (d), 65.6 (t), 44.7 (t), 43.5 (t), 42.2 (t), 35.7 (t), 34.3 (t), 33.5 (t), 30.9 (t), 28.7 (t), 19.6 (q).

MS-EI (m/z, %): 498 ($M^+$, 1.5), 480 (8), 462 (3.5), 395 (7), 217 (13), 179 (40), 81 (75). HRMS: found: 498.2969, $C_{30}H_{42}O_6$ requires 498.2981.

Laulimalide **7e**

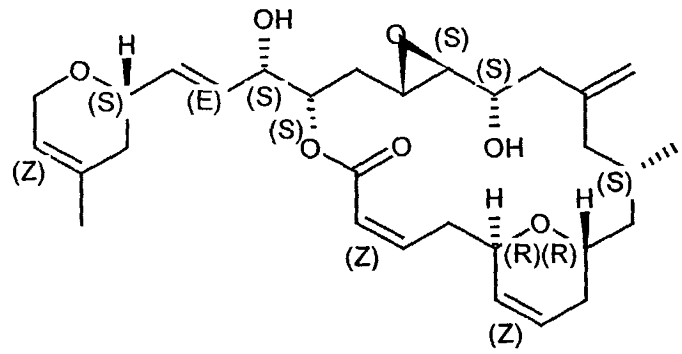

$C_{30}H_{42}O_7$
Exact Mass: 514,2931
Mol. Wt: 514,6503
C, 70,01; H, 8,23; O, 21,76

To a suspension of 4 A molecular sieves (50mg) in $CH_2Cl_2$ (2ml) at -20°C was added Ti (*i*PrO)$_4$, (3µl, 0.01 mmol). The mixture was stirred for 30min at -20°C and then *t*BuOOH (2µl, 5.5M, 0.01 mmol) was added. The mixture was stirred for additional 20 min and then a solution of compound **7d** (0.03mg, 0.01 mmol) in $CH_2Cl_2$ (1ml) was added. The resulting mixture was stirred for 2h at -20°C.
After this period a mixture of 4 N NaOH (0.2ml) and brine (1ml) was added and the resulting mixture was stirred at 0°C for 1h. The layers was separated and the aqueous phase was extracted with $CH_2Cl_2$. The combined organic layers were washed with brine, dried ($Na_2SO_4$) and concentrated. HPLC purification of the crude product (hexane: ethyl acetate- 1:1) gave **7e** as a colorless oil (0.02g), $[\alpha]_D^{20}$=-195° (c=0.01 in $CHCl_3$); $^1$H-NMR (600 MHz, $CDCl_3$); δ in ppm=6.46 (1H, td, *J*=10.3, 4.1 HZ), 5.88 (3H, m), 5.76 (1H, dd, *J*=15.6, 5.7, Hz), 5.70 (1H, brd, *J*=10.3), 5.41 (1H, brs), 5.15 (1H, dd, *J*=10.4, 4.4Hz), 4.86 (1H, brs), 4.84 (1H, brs), 4.30 (1H, m), 4.22 (1H, dd, *J*=6.0, 5.2Hz), 4.17 (2H, m), 4.07 (1H, m), 4.00 (1H, m). 3.75 (2H, m), 3.07 (1H, ddd, *J*=8.9, 3.4, 2.4Hz), 2.89 (1H, t, 2.4 Hz), 2.37 (2H, m), 2.23 (1H, ddd, *J*=16.6, 6.1, 3.5 Hz), 2.10 (1H, d, *J*=14.5), 2.0-1.81 (5H, m), 1.79 (1H, dd, *J*=12.5, 9.5 Hz), 1.7 (3H, brs), 15-1.4 (2H, m), 1.30 (1H, m), 0.80 (3H, d, *J*=6.6Hz); $^{13}$C-NMR (150 MHz, $CDCl_3$): δ=166.0 (s), 150.3 (d), 144.8 (s), 131.2 (s), 128.7 (d), 128.5 (d), 125.2 (d), 120.5 (d), 119.7 (d), 112.5 (t), 73.5 (d), 73.1 (d), 72.3 (d), 67.8 (d), 66.6 (d), 65.6 (t), 60.7 (d), 52.1 (d), 45.6 (t), 43.4 (t), 37.1 (t), 35.6 (t), 33.8 (t), 33.4 (t), 31.7 (t), 29.2 (d), 22.9 (d), 20.2 (q). MS HRMS: found: 514,2900, $C_{30}H_{42}O_7$ requires 514,2931.

**1.2.5 Description of the process for the production of intermediate II according to Scheme 2**

**i) Process for the production of compound 3**

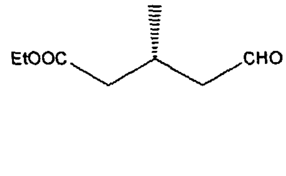

$C_8H_{14}O_3$
Exact Mass: 158,0943
Mol. Wt.: 158,1950
C, 60,74; H, 8,92; O, 30,34

A solution of alcohol compound **2** (0.8 g, 5 mmol) in dry $CH_2Cl_2$ (10 ml) was added dropwise to a slurry of Dess -Martin periodinane (6.5 g, 15 mmol) and $NaHCO_3$ (10 g) in dichloromethane ($CH_2Cl_2$) (40 ml). The mixture was stirred at room temperature for 1h, then subjected directly to $SiO_2$ Flash chromatography (hexane-ethyl acetate :5:1) to yield compound **3** (0.77g, 98%) as a colourless liquid; $[\alpha]_D^{20}$= -5.5° (c= 0.04, $CHCl_3$); IR (neat) 2954, 2915, 1730,

1717, 1415, 1360, 1238, 1182, 940 $cm^{-1}$; $^1$H-NMR (250 MHz, $CDCl_3$); δ in ppm = 9.80 (1H, s), 4.25 (2H, q, *J*=7.5 Hz), 3.75 (3H, m), 2.8-2.2 (5H, m), 1.22 (3H, t, *J*=7.5 Hz), 1.05(3H, d, *J*=7.2 Hz); $^{13}$C-NMR (62.5 MHz, $CDCl_3$), δ in ppm = 204.0 (d), 168.2 (s), 60.8 (t), 50.4 (t), 41.4 (t), 25.7 (d), 20.4 (q), 14.6 (q); HRMS: found 158,0450 $^+$. 5 ppm, $C_8H_{14}O_3$ requires 158,0943.

**ii) Process for the production of compound 4**

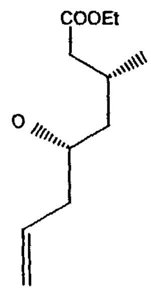

$C_{11}H_{20}O_3$
Exact Mass: 200,1412
Mol. WT.: 200,2747
C, 65,97; H, 10,07; O, 23,97

Aldehyde compound **3** of step **i)** (0.6 g, 3.8 mmol) was dissolved in dry $Et_2O$ (20 ml) and cooled to -100°C. To this solution was added (-) $Ipc_2BAII$ (9 ml, 0.5 M in pentane, 1.2 eqv) by cannulation during 30 min. After the completion of the addition, the mixture was stirred at the same temperature for 30 min. Methanol (1 ml) was added at -100°C and the reaction mixture was allowed to reach room temperature. The solvent was evaporated and the residue was dissolved in THF (20 ml). To this solution was added a solution of sodium perborate (1g in 10 ml water,) and the mixture was stirred for 30 min. The organic phase was separated and the water phase was extracted with ethyl acetate (3 X 5 ml). The combined organic layers were washed with water, brine, dried ($Na_2SO_4$) and concentrated. Flash chromatography of the crude mixture (ethyl acetate :hexane -5:1) provided **4**.(0.66 g, 87%), oil, $[\alpha]_D^{20}$= -9.4° (c= 0.65, $CHCl_3$); IR (neat) 3460, 2925, 1730, 1633, 1499, 1460, 1397, 1262, 809 $cm^{-1}$; $^1$H-NMR (250 MHz, $CDCl_3$); δ in ppm = $^1$H-NMR (250 MHz, $CDCl_3$); δ in ppm = 5.86 (1H, m), 5.11 (2H, brd, *J*=14.0 Hz), 4,16 (2H, q, *J*=7.0 Hz), 3.75 (1H, brd, $w_{1/2}$ = 17Hz), 2.4-1.9 (5H, m), 1.45 (1H, m), 1.32 (1H, m), 1.24 (3H, t, *J*=7.0, Hz), 0.99 (3H, d, *J*=6.5, Hz); $^{13}$C-NMR (62.5 MHz, $CDCl_3$), δ in ppm =173.5 (s), 132.9 (d), 118.9 (t), 71.6 (d), 60.6 (t), 44.0 (t), 43.2 (t), 42.6 (t), 27.6 (d), 19.9.4 (q), 14.6 (q); 27.6 (d), 19.9.4 (q), 14.6 (q); MS-EI (m/z, %): 200 ($M^+$, 0.3), 182 ($M^+$ - $H_2O$, 20), 172 (25), 158 (20), 128 (12), 85 (83), 72 (100); HRMS: found 1821, 1310 $^+_-$ 5 ppm, $C_{11}H_{18}O_2$ ($M^+$-$H_2O$) requires 182,1307.

**iii) Process for the production of compound 5**

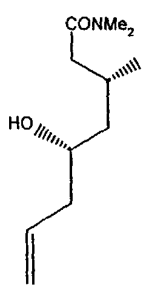

$C_{11}H_{21}NO_2$
Exact Mass: 199,1572
Mol. Wt.: 199,2900

C, 66,29; H, 10,62; N, 7,03; O, 1606

A solution of alcohol compound **4** of step **ii)** (0.6 g, 3mmol) in dimethylamine (5.6 M in ethanol, 10 ml) was heated for 2h in sailed flask at 40°C and then, was allowed to stay 12 h at room temperature. The ethanol was removed and the residue was filtered trough $SiO_2$, ($CH_2Cl_2$ : MeOH - 97:3) to give pure amide **5** (0.58 g, 98%) as a yellow oil; $[\alpha]_D^{20}$= -9.4° (c= 0.65 in $CHCl_3$).); IR (neat) 3665, 3645, 3400, 2925, 1633, 1504, 1403, 1056, $cm^{-1}$; $^1$H-NMR (250 MHz, $CDCl_3$); δ in ppm = 5.86 (1H, m), 5.11 (2H, brd, *J*=14.0 Hz), 3.01 (3H, s), 2.96 (3H, s), 3.75 (1H, brs, $w_{1/2}$ = 19Hz), 2.4-2.2 (5H, m), 1.45 (2H, m), 0.99 (3H, d, *J*=6.5, Hz); $^{13}$C-NMR NMR (62.5 MHz, $CDCl_3$), δ in ppm =175.5 (s), 135.6 (d), 117.8 (t), 69.7 (d), 44.6 (t), 43.3 (t), 41.3 (t), 37.7 (q), 35.8 (q), 27.6 (d), 21.2 (q); MS-EI (m/z, %): 199 ($M^+$, 0.5), 188 ($M^+$ - $H_2O$, 15), 172 (20), 158 (32), 128 (12), 95 (23), 85 (83), 72 (100); HRMS: found 181,1470 $^+_-$ 5 ppm, $C_{11}H_{19}O_2N$ ($M^+$-$H_2O$) requires 181,1466.

**iv) Process for the production of compound 6**

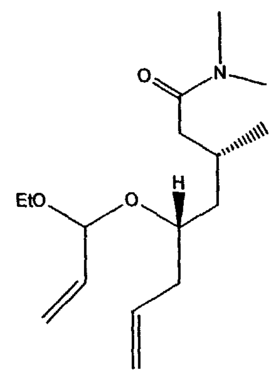

$C_{16}H_{29}NO_3$
Exact Mass: 283,2147
Mol. Wt.: 283,4064
C, 67,81; H, 10,31; N, 4,94; O, 16,94

To a solution of compound **5** of step **iii)** (0.350 g, 1.7 mmol) and acrolein-diethylacetal (0.38 ml, 2.4 mmol) in dry toluene (30 ml) was added TsOH (10 mg) and the mixture was slowly rotated on a rotavapour at 80°C mbars and 40°C. The reaction was monitored by TLC and the volume of the solvent was kept constant by addition of toluene. At the end of the reaction, the mixture was washed with $NaHCO_3$, water, brine, dried ($Na_2SO_4$) and concentrated. Purification by Flash chromatography (ethyl acetate : toluene - 3:1) provided compound **6** (0.46 g, 92%), as a mixture of diastereomers (1:1); IR (neat) 3041, 3077, 2925, 1651, 1451, 1497, 1324, 1262, 1181, 1103 $cm^{-1}$; $^1$H-NMR (250 MHz, $CDCl_3$); δ in ppm = 5.81 (4H, m), 5,40 (2H, d, *J*=16.2, Hz), 5,28 (2H, d, *J*=12.6, Hz), 5.10 (4H, m), 4.97 (1H, d, *J*=5.3, Hz), 4.91 (1H, d, *J*=5.5, Hz), 3.50 (4H, m), 3.01 (6H, s), 2.94 (6H, s), 2.50-1.56 (12H, m), 1.37 (2H, m), 1.17 (6H, t, *J*=7.0 Hz) 1.0 (3H, d, *J*=6.2 Hz ), 0.95 (3H, d, *J*=6.2 Hz); $^{13}$C-NMR (62.5 MHz, $CDCl_3$), δ in ppm = 172.7 (s), 172.6 (s) 136.4 (d), 136.3 (d), 135.3 (d), 134.9 (d), 118.3 (t), 118.2 (t), 117.6 (t), 117.5 (t), 102.4 (d), 100.0 (d), 75.00 (d), 75.55 (d) 60.8 (t) 60.7 (t), 42.5 (t), 42.2 (t), 41.6 (t), 41.5 (t), 40.4 (t),39.5 (t), 37.9 (q), 37.7 (q), 35.7 (q), 27.2 (d), 20.6 (q), 20.3 (q), 15.6 (q), 15.5 (q); HRMS: found 283,2155. $^+_-$ 5 ppm, $C_{16}H_{29}NO_3$ requires 283,2147

**v) Process for the production of compound 7**

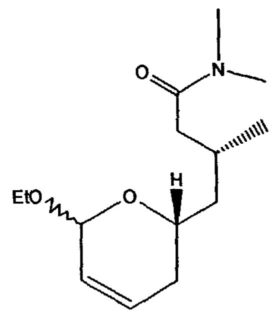

$C_{14}H_{25}NO_3$
Exact Mass: 255,1834
Mol. Wt.: 255,3532
C, 67,85; H, 9,87; N, 5,49; O, 18,80

A solution of compound **6** of step **iv)** (0.42 g, 1.5 mmol) and Grubs catalyst (0.06 g, 0.007 mmol) in $CH_2Cl_2$ was deoxygenated by a freeze-thaw cycle in which the solvent was first frozen in liquid $N_2$, and then warmed up in vacuo. The deoxygenated mixture was refluxed for a 6 h (TLC monitoring). At the end of the reaction the catalyst was destroyed by passing an air through the solution for 15 min. The solvent was evaporated and the crude product was purified by Flash chromatography (ethyl acetate : toluene : TEA-3:1.0.1) to give **7** as a mixture of diastereomers (1:1) (0.35 g, 92%), oil; IR (neat) 3041, 2925, 1644, 1455, 1395, 1324, 1262, 1181, 1103 $cm^{-1}$; $^1$H-NMR (250 MHz, $CDCl_3$); δ in ppm = 5.95 (2H, m), 5,75 (1H, d, *J*=10.6, Hz), 5,66 (1H, d, *J*=10.6, Hz), 5.04 (1H, brs), 4.96 (1H, brs), 3.90 (4H, m), 3.54 (2H, m), 3.01 (6H, s), 2.94 (6H, s), 2.50-1.80 (12H, m), 1.65 (2H, m) 1.32 (2H, m), 1.2 (6H, t, *J*=6.9 Hz) 1.0 (3H, d, *J*=6.2 Hz), 0.95 (3H, d, *J*=6.2 Hz); $^{13}$C-NMR (62.5 MHz, $CDCl_3$), δ in ppm = 172.7 (s), 172.6 (s) 129.4 (d). 128.1 (d), 126.0 (d), 98.1 (d), 94.8 (d), 70.69 (d), 64.4 (d), 64.0 (t), 63.5 (t), 43.1 (t), 43.0 (t),41.4 (t) 37.9 (q), 35.7 (q), 31.7 (t), 31.5 (t), 27.5 (d), 26.9 (d), 20.5 (q), 19.8 (q), 15.7 (q); HRMS: found 255,1840 $^{+}_{-}$ 5 ppm, $C_{14}H_{25}NO_3$ requires 255,1834.

**vi) Process for the production of compound 8**

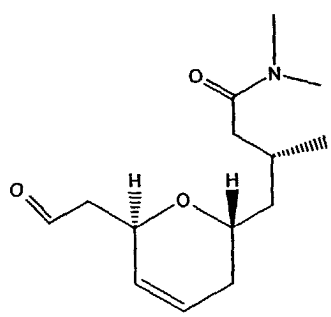

$C_{14}H_{23}NO_3$
Exact Mass: 253,1678
Mol. Wt.: 253,3374
C, 66,37; H, 9,15; N, 5,53; O, 18,95

A mixture of compound **7** of step **v)** (0.12 g, 0.47 mmol), vinyl-TBS ether (0.1 g, 0.62mmol) and a solution of $LiClO_4$ in ethyl acetate (5 ml, 3M) was kept at room temperature for 24 h. The mixture was diluted with water (10 ml) and the organic phase was separated. The water phase was extracted with $CHCl_3$ (3 X 5 ml). The combined organic layers were washed with water, brine, dried ($Na_2SO_4$) and concentrated. Purification by Flash chromatography (ethyl acetate : toluene -3:1) provided **8** (0.105 g, 89%), oil, $[\alpha]_D^{20}$= -8.8° (c= 0.5 in $CHCl_3$); IR (neat) 2925, 1724, 1633, 1499,

1460, 1461, 1397, 1262, 809 $cm^{-1}$; $^1H$-NMR (250 MHz, $CDCl_3$); δ in ppm = 9.79 (1H, dd, *J*=3, 1.7 Hz), 5.75 (1H, ddd, *J*=10.3, 5, 2.5 Hz), 5,68 (1H, ddd, *J*=10.3, 4, 2.5 Hz), 4.90 (1H, m), 3.78 (1H, m), 2.98 (3H, s), 2.82 (3H, s), 2.70 (1H, ddd, *J*=16.0, 8.8, 3 Hz), 2.52 (1H, ddd, *J*=16, 4.7, 1.7 Hz), 2.2-2.1 (4H, m), 1.9 (1H, m), 1.80 (1H, m), 1.10 (1H, m), 0.90 (3H, d, *J*=6.2 Hz); $^{13}C$-NMR (62.5 MHz, $CDCl_3$), δ in ppm = 201.4 (d), 172.6 (s), 128.2 (d), 125.7 (d), 67.8 (d), 66.4 (d), 48.4 (t), 41.4 (t), 37.9 (q), 35.8 (q), 31.0 (t), 26.9 (d), 20.2 (q); MS-EI (m/z, %): 253 (M+, 8), 238 (3), 180 (15), 156 (9), 126 (12), 95 (15), 87 (100); HRMS: found 253,1662 $^+_-$ 5 ppm, $C_{14}H_{23}NO_3$ requires 253,1678.

**vii) Process for the production of compound 9**

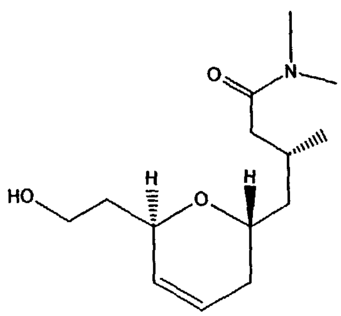

$C_{14}H_{25}NO_3$
Exact Mass: 255,1834
Mol. Wt.: 255,3532
C, 66,85; H, 9,87; N, 5,49; O, 18,80

Reduction of aldehyde compound **8** of step **vi)** (0.140 g, 0.55mmol) was performed in MeOH (3ml) with $NaBH_4$ (0.015g) at 0°C. The reaction was quenched with 0.5 ml saturated solution of $NH_4Cl$, the solvent was evaporated and the mixture was extracted with ethyl acetate (3 X 5 ml). The combined extracts were washed with water followed by brine, dried ($Na_2SO_4$) and concentrated to give pure alcohol of compound **9** (0.14 g) which was used in the next step without further purification. IR (neat) 3650, 3500, 3030, 2953, 2876, 1653, 1495, 1460, 1415, 1394, 1263, 1089 $cm^{-1}$; $^1H$-NMR (250 MHz, $CDCl_3$); δ in ppm = 5.75 (1H, brd, *J*=10.3 Hz), 5,65 (1H, brd, *J*=10.3 Hz), 4.36 (1H, brd, *J*=9.8 Hz), 3.70 (3H, m), 2.98 (3H, s), 2.82 (3H, s), 2.3-2.1 (4H, m), 1.9-1.5 (4H, m), 1.20 (1H, m), 0.94 (3H, d, *J*=6.4 Hz); $^{13}C$-NMR (62.5 MHz, $CDCl_3$), δ in ppm = 172.6 (s), 129.9 (d), 124.3 (d), 70.1 (d), 66.4 (d), 60.4 (t), 42.1 (t), 41.4 (t), 37.8 (q), 36.7 (t), 35.8 (q), 31.0 (t), 27.0 (d), 20.3 (q), MS-EI (m/z, %): 255 (M+, 8), 237 (3), 210 (28), 182 (15), 158 (16), 140 (12), 128 (14), 114 (17), 95 (15), 87 (100), 72( 84); HRMS: found 255,1816 $^+_-$ 5 ppm, $C_{14}H_{25}NO_3$ requires 255,1834.

**viii) Process for the production of compound 10**

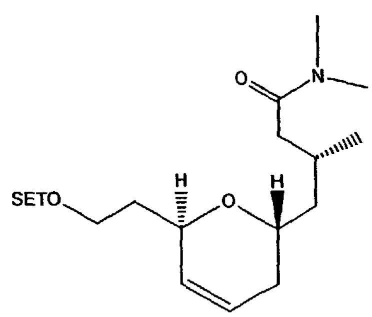

$C_{20}H_{39}NO_3Si$

Exact Mass: 255,1834
Mol. Wt.: 369,6141
C, 64,99; H, 10,64; N, 3,79; O, 12,99; Si, 7,60

To a solution of compound **9** of step **vii)** (0.13 g, 0.5 mmol) in pyridine (5 ml) was added TESCI (0.12 ml, 0.7 mmol) and the mixture was stirred at room temperature for 30 min. The mixture was diluted with toluene (10 ml), washed with water, brine, dried ($Na_2SO_4$) and concentrated. The crude product was purified by Flash chromatography (hexane : ethyl acetate - 1:1) to give compound **10** (0.18 g, 98 %), oil, $[\alpha]_D^{20}$= -20.0° (c= 0.60, $CHCl_3$); IR (neat) 3030, 2953, 2915, 2876, 1653, 1495, 1460, 1415, 1394, 1263, 1089 $cm^{-1}$; $^1$H-NMR (250 MHz, $CDCl_3$); δ in ppm = 5.76 (1H, brd, *J*=10.3 Hz), 5,65 (1H, brd, *J*=10.3 Hz), 4.28 (1H, m), 3.70 (3H, m), 2.98 (3H, s), 2.90 (3H, s), 2.3-2.1 (3H, m), 1.9-1.5 (5H, m), 1.20 (1H, m), 0.94 (3H, d, *J*=6.4 Hz), 0.90 (9H, t, *J*=7.5 Hz), 0.60 (6H, q, *J*=7.5 Hz); $^{13}$C-NMR (62.5 MHz, $CDCl_3$), δ in ppm = 172.6 (s), 130.2 (d), 124.3 (d), 68.7 (d), 65.6 (d), 59.9 (t), 43.1 (t), 41.6 (t), 37.8 (q), 37.4 (t), 35.6 (q), 31.7 (t), 27.2 (d), 20.1 (q), 7.1 (q), 4.7 (t); MS-EI (m/z, %): 369 (M+, 2), 340 (8), 255 (5), 210 (10), 182 (7), 132 (7), 103 (100), 75 (85); HRMS: found 369,2670 $^+_-$ 5 ppm, $C_{20}H_{39}NO_3Si$ requires 369,2699.

**ix) Process for the production of compound 11**

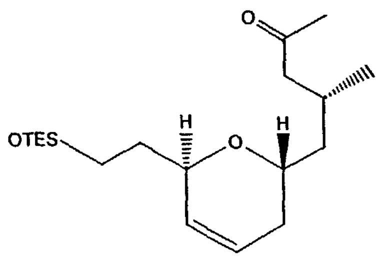

$C_{19}H_{36}O_3Si$
Exact Mass: 340,2434
Mol. Wt.: 340,5728
C, 67,01; H, 10,65; O, 14,09; Si, 8,25

To a solution of amide compound **10** of step **ix)** (0.2 g, 0.54 mmol) in $Et_2O$ (8 ml) was added a solution of MeLi (0.44 ml, 1.6M in $Et_2O$, 0.7 mmol) at -78°. The mixture was stirred for 0.5 h and then the cooling bath was removed and the mixture was allowed to warm up to room temperature. The reaction was quenched with saturated solution of $NH_4Cl$, the organic phase was washed with water, brine, dried ($Na_2SO_4$) and concentrated. The crude product was purified by Flash chromatography (hexane : ethyl acetate -5:1) to give compound **11** (0.17 g, 94 %) oil, $[\alpha]_D^{20}$= -30.0° (c= 0.40, $CHCl_3$); IR (neat) 2954, 2915, 2876, 1717, 1654 1459, 1415, 1360, 1238, 1182, 1090, 940 $cm^{-1}$; $^1$H-NMR (250 MHz, $CDCl_3$); δ in ppm = 5.80 (1H, brd, *J*=11.6 Hz), 5,71 (1H, brd, *J*=11.6 Hz), 4.35 (1H, m), 3.75 (3H, m), 2.47 (1H, dd, *J*=18.0, 9.0 Hz), 2.28 (1H, dd, *J*=18.0, 7.5 Hz), 2.1 (3H, s), 1.8-1.5 (6H, m), 1.20 (1H, m), 0.95 (12H, t, *J*=7.5 Hz), 0.60 (6H, q, *J*=7.5 Hz); $^{13}$C-NMR (62.5 MHz, $CDCl_3$); δ in ppm = 208.0 (s), 130.3 (d), 124.3 (d), 68.8 (d), 65.4 (d), 59.9 (t), 52.3 (t), 43.0 (d), 37.3 (t), 31.7 (t), 30.3 (q), 26.4 (d), 19.2 (q), 7.1 (q), 4.7 (t); MS-EI (m/z, %): 340 (M+, 3), 311 (100), 281 (12), 269 (7), 213 (80), 183 (43), 150 (38), 109 (70), 81 (65); HRMS: found 340,2439 $^+_-$ 5 ppm, $C_{14}H_{25}NO_3$ requires 340,2434.

**x) Process for the production of compound 12**

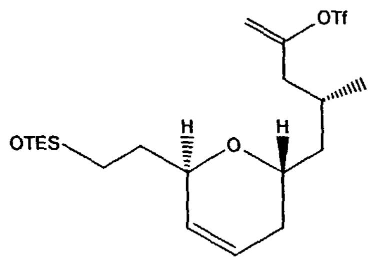

$C_{20}H_{35}F_3O_5SSi$
Exact Mass: 340,2434
Mol. Wt.: 472,1827
C, 50,82; H, 7,46; F, 12,06; O, 16,93; S, 6,78; Si, 5,94

To a solution of KHMDS (3 ml, 0.5 M in toluene, 1.5 mmol) was added slowly a solution of ketone compound **11** of step **ix)** (0.4 g, 1.2 mmol) in THF (5 ml) at -78°C. The mixture was stirred for 1 h and then a solution of PhN $(OTf)_2$, (0.59 g, 1.6 mmol) in THF (1ml) was added at same temperature. After being stirred for additional 1 h at -78°C, the cooling bath was removed and the mixture was allowed to warm up to room temperature. The reaction was quenched with saturated solution of $Na_2CO_3$ (1 ml) and the solution was diluted with toluene (20 ml).The organic phase was washed with saturated solution of $Na_2CO_3$ (2 X 2 ml), water, brine, dried ($Na_2SO_4$) and concentrated. The crude product was purified by Flash chromatography (hexane: ethyl acetate: TEA-20:1:0.1) to give compound **12** (0.38 g, 68%) oil, $[\alpha]_D^{20}$= -34.8° (c= 0.42, $CHCl_3$); IR (neat) 2956, 2937, 2877, 1731, 1668, 1574, 1537, 1504, 1460, 1248, 1212, 1182, 1097, 906 $cm^{-1}$; $^1$H-NMR (250 MHz, $CDCl_3$); δ in ppm = 5.82 (1H, brd, *J*=11.6 Hz), 5,73 (1H, brd, *J*=11.6 Hz), 5.17 (1H, d, *J*=3.4 Hz) 5.05 (1H, d, *J*=3.4 Hz),

**xi) Process for the production of compound 13**

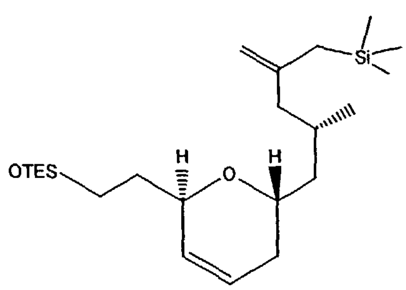

$C_{23}H_{46}O_2SSi_2$
Exact Mass: 410,3036
Mol. Wt.: 410,7811
C, 67,25; H, 11,29; O, 7,79; Si, 13,67

A slurry of $Pd(Ph_3P)_4$ (0.018 g, 0.015 mmol) and LiCl (0.03 g, 0.04 mmol) in ether (15 ml) was stirred for 10 min and then a solution of enoltriflate **12** of step **x)** (0.1 g, 0.2 mmol) in ether (1 ml) was added dropwise. After being stirred for additional 10 min a solution of $TMSCH_2MgCl$ (0.4 ml, 1M in ether, 0.4 mmol) was added at once. After 15 min, the reaction was quenched with saturated solution of $Na_2CO_3$ (1 ml) and the solution was diluted with toluene (10 ml). The organic phase was washed with water followed by brine, dried ($Na_2SO_4$) and concentrated. The crude product was purified by Flash chromatography (hexane: ethyl acetate : TEA-25:1:0.1) to give compound **13** (0.084 g, 96%) oil, $[\alpha]_D^{20}$= -38.7° (c= 0.40, $CHCl_3$); IR (neat) 3071, 3032, 2954, 1631, 1418, 1392, 1247, 1180, 1158, 1097, 850 $cm^{-1}$; $^1$H-NMR (250 MHz, $CDCl_3$); δ in ppm = 5.85 (1H, brd, *J*=11.4 Hz), 5,71 (1H, brd, *J*=11.4 Hz), 4.60 (2H, ABq,

*J*=8.3 Hz), 4.33 (1H, brd, *J*=9.3 Hz), 3.82 (3H, m), 2.0-1.8 (6H, m), 1.7-1.5 (3H, m), 1.10-1.0 (2H, m), 0.92 (9H, t, *J*=7.9 Hz), 0.86 (3H, d, *J*=6.6,), 0.65 (6H, q, *J*=7.9 Hz), 0.17 (9H, s); $^{13}$C-NMR (62.5 MHz, $CDCl_3$), δ in ppm = 201.37 (d), 146.4 (s), 128.2.4 (d), 126.1 (d), 109.2 (t), 68.2 (d), 66.0 (d), 48.3 (t), 47.2 (t), 42.5 (t), 31.4 (t), 27.1 (d), 26.7.0 (t), 19.7 (q), 0.8 (q); MS-EI (m/z, %): 410 ($M^+$, 8), 395 (6), 278 (27), 239 (84), 181 (25), 155 (22), 117 (38), 73 (100); HRMS: found 410,3021 $^+_-$ 5 ppm, $C_{23}H_{46}O_2Si_2$ requires 410,3036.

**xii) Process for the production of compound 14**

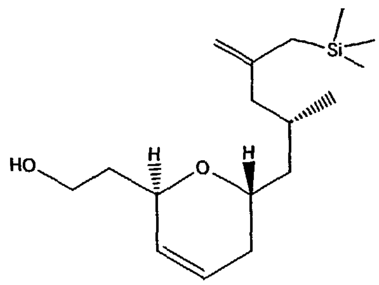

$C_{17}H_{32}O_2Si$
Exact Mass: 296,2172
Mol. Wt.: 296,5203
C, 68,86; H, 10,88; O, 10,79; Si, 9,47

The slurry $K_2CO_3$ (0.2 g) and compound **13** of step **xi)** (0.1g) in methanol (2 ml) was stirred for 12 h at room temperature. The solvent was evaporated and saturated solution of $NH_4Cl$ (1 ml) was added. The mixture was extracted with ethyl acetate (3 X 5 ml). The organic phase was washed with water followed by brine, dried ($Na_2SO_4$) and concentrated. The crude mixture was purified by Flash chromatography (hexane :ethyl acetate :TEA-10:1:0.1) to give the alcohol **14** (0.07 g, 96%), oil; $[\alpha]_D^{20}$= -42.5° (c= 0.50, $CHCl_3$); IR (neat) 3540, 3071, 3030, 2960, 1631, 1418, 1392, 1180, 1158, 850 cm$^{-1}$; $^1$H-NMR (250 MHz, $CDCl_3$); δ in ppm = 5.82 (1H, brd, *J*=11.4 Hz), 5,72 (1H, brd, *J*=11.4 Hz), 4.57 (2H, s), 4.30 (1H, brd, *J*=9.3 Hz), 3.82 (3H, m), 2.0-1.8 (6H, m), 1.7-1.5 (3H, m), 1.10-1.0 (2H, m), 0.91 (3H, d, *J*=6.6,), 0.17 (9H, s); $^{13}$C-NMR (62.5 MHz, $CDCl_3$), δ in ppm = 146.0 (s), 130.4 (d), 124.8 (d), 110.0 (t), 69.9 (d), 65.2 (d), 47.3 (t), 43.3 (t), 37.2 (t), 32.0 (t), 27.2 (d), 26.6 (t), 19.6 (q), 4.72 (t), -0.9 (q); HRMS: found 296,2175 $^+_-$ 5 ppm, $C_{17}H_{32}O_2Si$ requires 296,2172.

**xiii) Process for the production of compound 15**

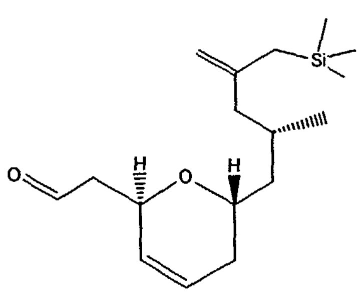

$C_{17}H_{30}O_2Si$
Exact Mass: 294,2015
Mol. Wt.: 294,5044
C, 69,33; H, 10,27; O, 10,87; Si, 9,54

To a mixture of alcohol (0.05 g, 0.17 mmol) and TEA (0.07 ml) in $CH_2Cl_2$ (1.5 ml) was added a solution of $SO_3$.Py complex (0.08 g, 0.5 mmol) in DMSO (1.5 ml) at 0°C. and stirred for 15 min. The reaction was quenched with

saturated solution of $NH_4Cl$, and extracted with ethyl acetate (3 X 5 ml). The combined extracts were washed with water followed by brine, dried ($Na_2SO_4$) and concentrated. Flash chromatography (hexane : ethyl acetate:TEA-15:1: 0.1) of the crude products gave compound **15** (0.044 g, 86%), oil, $[\alpha]_D^{20}$= -65.3° (c= 0.68, $CHCl_3$); IR (neat) 2952, 2924, 1725, 1647, 1438, 1248, 1216,1168, 1093 $cm^{-1}$; $^1$H-NMR (250 MHz, $CDCl_3$); δ in ppm = 9.81 (1H, dd, *J*=3.1, 1.7), 5.90 (1H, m), 5.74 (1H, d, *J*=11.9), 4.80 (1H, m), 4.60 (2H, brs), 3.79 (1H, m), 2.84 (1H, ddd, *J*=16.0, 9.0, 3.0), 2.60 (1H, ddd, *J*=16.0, 4.6, 1.8), 2.0-1.8 (5H, m), 1.7-1.5 (2H, m), 1.10 (1H, m), 09 (3H, d, *J*=6.4,), 0.04 (9H, s); $^{13}$C-NMR (62.5 MHz, $CDCl_3$), δ in ppm = 168.8 (s), 147.7 (d), 146.5 (s), 129.4 (d), 125.4 (d), 120.8 (d), 109.1 (t), 72.3 (d), 65.7 (d), 51.4 (q), 47.3 (t), 42.9 (t), 33.9 (t), 31.7 (t), 27.2 (d), 26.7 (t), 19.7 (q), -0.8 (q); HRMS: found: 294,2010 $^{+}_{-}$ 5 ppm, $C_{17}H_{30}O_2Si$ requires 294,2015.

**xiv) Process for the production of compound 16**

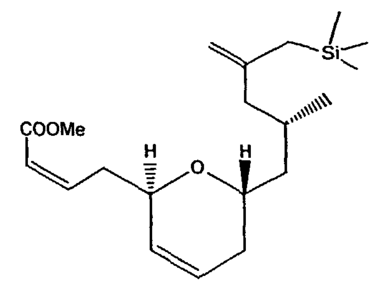

$C_{20}H_{34}O_3Si$
Exact Mass: 350,2277
Mol. Wt.: 350,5677
C, 68,52; H, 9,78; O, 13,69; Si, 8,01

To a mixture of $(CF_3CH_2O)_2P(O)CH_2COOMe$ (0.1 g, 0.3 mmol) and 18-crown-6 (0.43 g, 1,6 mmol) in THF (2 ml) was added KHMDS (0.68 ml, 0.5 M in Tol, 0.3 mmol) at -78°C. After 45 min a solution of aldehyde compound **15** (0.08 g, 0.28 mmol) in THF (1 ml) was added dropwise and the mixture was stirred additional 45 min. The reaction was quenched with saturated solution of $NH_4Cl$ and extracted with ethyl acetate, (3 X 5 ml). The combined extracts were washed with water followed by brine, dried ($Na_2SO_4$) and concentrated. Flash chromatography of the crude products (ethyl acetate : hexane 1:1) gave pure (Z)- isomer (0.085 g, 90%) as a colourless oil, $[\alpha]_D^{20}$= -68.6° (c= 0.23, $CHCl_3$); IR (neat) 2952, 2924, 1725, 1647, 1438, 1248, 1216, 1168, 1093 $cm^{-11}$H-NMR (250 MHz, $CDCl_3$); δ in ppm = 6.41 (1H, ddd, *J*=11.6, 7.3, 4.3), 5.85 (2H, m), 5,75 (1H, brd, *J*=11.4), 4.58 (2H, brs), 4.30 (1H, brs), 3.90 (1H, m), 3.81 (3H, s), 2.98 (2H, m), 2.0-1.8 (5H, m), 1.7-1.5 (2H, m), 1.10 (1H, m), 0.86 (3H, d, *J*=6.6,), 0.17 (3H, s); $^{13}$C-NMR (62.5 MHz, $CDCl_3$); δ in ppm = 168.8 (s), 147.7 (d), 146.5 (s), 129.4 (d), 125.4 (d), 120.8 (d), 109.1 (t), 72.3 (d), 65.7 (d), 51.4 (q), 47.3 (t), 42.9 (t), 33.9 (t), 31.7 (t), 27.2 (d), 26.7 (t), 19.7 (q), -0.8 (q); MS-EI (m/z, %): 350 ($M^+$, 4), 335 (3), 251 (55), 182 (38), 109 (13), 73 (100); HRMS: found: 350,2273 $^{+}_{-}$ 5 ppm, $C_{20}H_{34}O_3Si$ requires 350,2260.

**xv) Process for the production of compound 2a**

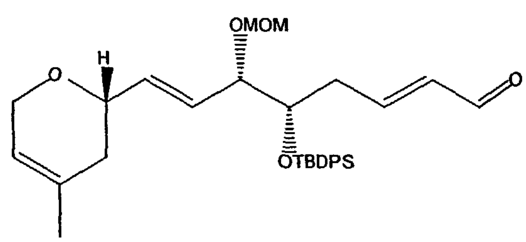

$C_{32}H_{42}O_5Si$
Exact Mass: 534,2802

Mol. Wt.: 534,7584

C, 71,87; H, 7,92; O, 14,96; Si, 5,25

A solution. of alcohol compound **1a** (0.53 g, 1 mmol) in dry $CH_2Cl_2$ (2 ml) was added dropwise to a slurry of Dess -Martin periodinane (1.3 g, 5 mmol) and $NaHCO_3$ (2 g) in $CH_2Cl_2$ (20 ml). The mixture was stirred at room temperature for 15 min, then subjected directly to $SiO_2$ Flash chromatography (hexane-ethyl acetate : 5:1) to yield compound **2a** (0.500g, 95%) as a colourless liquid, $[\alpha]_D^{20}$= -9.7° (c= 0.75, $CHCl_3$); IR (neat) 29312, 2924, 1692, 1427, 1106, 1033, 977, 741 $cm^{-1}$ $^1$H-NMR (250 MHz, $CDCl_3$); δ in ppm =9.28 (1H, d, , *J*=7.9 Hz), 7.71 (4H, m), 7.43 (6H, m), 6.64 (1H, ddd, *J*=15.4, 7.4, 7.4 Hz), 5.94 (1H, dd, *J*=15.4, 7.9 Hz), 5.81 (2H, m), 5.45 (1H, brs), 4.54 (1H, d, *J*=6.6 Hz), 4.44 (1H, d, *J*=6.6 Hz), 4.13 (5H, m), 3.20 (3H, s), 2.47 (2H, m), 1.75 (3H, brs), 1.10 (9H, s); $^{13}$C-NMR (62.5 MHz, $CDCl_3$), δ in ppm =194.3 (d), 156.2 (d), 136.4 (d), 136.3 (d), 135.1 (d), 134.7 (s), 131.7 (s), 128.1 (d), 126.6 (d), 120.2 (d), 95.1 (t), 78.8 (d), 65.9 (t), 55.9 (d), 36.3 (t), 36.0 (t), 27.4 (q), 23.3 (q), 21.4 (s); MS-EI (m/z, %): 534. ($M^+$, 0.1), 477 ($M^+$- *t*-Bu, 0.43), 415 (6), 337 (38), 109 (15), 267 (12), 233 (30), 199 (100), 135 (88), 91(30); HRMS: found: 477,2079 $^+_-$ 5 ppm, $C_{28}H_{33}O_5Si$ requires 477,2097 ($M^+$- *t*-Bu).

**xvi) Process for the production of compound 3a**

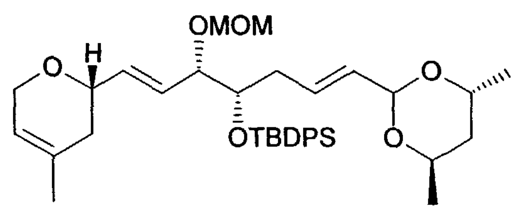

$C_{37}H_{52}O_6Si$

Exact Mass: 620,3533

Mol. Wt.: 620,8907

C, 71,57; H, 8,44; O, 15,46; Si, 4,52

To a solution of compound **2a** from step **xv)** ( 0.530 g, 1 mmol) and (R, R)-(-)-2,4-pentanediol (0.16 g, 1.6 mmol) in dry toluene (20 ml) was added Montmorillonit K-10 (20 mg) and the mixture was slowly rotated on a rotavapour at 80 mbars and 40°C. The reaction was monitored by TLC and the volume of the solvent was kept constant by addition of additional portions of solvent. At the end the reaction mixture was filtered trough a short pad of $SiO_2$ (hexane : ethyl acetate : TEA - 5:1:0.1) to give compound **3a** (0.55 g, 89%), oil, $[\alpha]_D^{20}$= +14.3° (c= 0.18, $CHCl_3$); IR (neat) 2940, 2925, 1427, 1130, 1125, 1106, 1033, 977, 741 $cm^{-1}$; $^1$H-NMR (250 MHz, $CDCl_3$); δ in ppm =7.71 (4H, m), 7.43 (6H, m), 5.78 (3H, m), 5.40 (2H, m), 5.19 (1H, d, *J*=5.0 Hz), 4.54 (1H, d, *J*=6.6 Hz), 4.40 (1H, d, *J*=6.6 Hz), 4.32 (1H, ddd *J*=13.2, 6.8, 6.6 Hz), 4.20 (2H, brs), 4.1-3.8 (4H, m), 3.20 (3H, s), 2.4-1.8 (5H, m), 1.83 (3H, brs), 1.4 (3H, d, *J*=7.2 Hz), 1.3 (2H, m), 1.24 (3H, d, *J*=6.4 Hz), 1.10 (9H, s); $^{13}$C-NMR (62.5 MHz, $CDCl_3$), δ in ppm =136.6 (d), 136.5 (d), 136.4 (d), 135.1 (d), 134.7 (s), 133.8 (s), 132.2 (d), 132.0 (s), 129.6 (d), 128.1 (d), 128.0 (d), 127.9 (d), 120.1 (d), 93.8 (t), 92.5 (d), 78.6 (d), 76.2 (d), 75.2 (d), 68.4 (d), 68.3 (d), 65.9 (t), 55.9 (d), 35.9 (t), 35.7 (t), 27.5 (q), 23.4 (q), 22.3 (q), 19.8 (t), 19.6 (s), 17.6 (q); MS-EI (m/z, %): 620 ($M^+$, 3), 563 (15), 423 (60), 337 (18), 275 (45), 199 (100), 135 (94); HRMS: found: 620,3541 $^+_-$ 5 ppm, $C_{37}H_{52}O_6Si$ requires 620,3533.

**xvii) Process for the production of compound 4a**

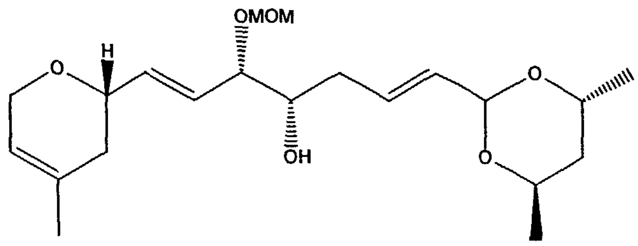

$C_{21}H_{34}O_6$
Exact Mass: 382,2355
Mol. Wt.: 382,4911
C, 65,94; H, 8,96; O, 25,10

A solution of compound **3a** of step **xvi)** (0.76, 1.2 mmol) and TBAF (1M, 3 ml) in THF (5 ml) was kept for 24 h at room temperature. The reaction was quenched with saturated solution of $NH_4Cl$. The organic phase was extracted with ethyl acetate, (3 X 5 ml). The combined extracts were washed with water followed by brine, dried ($Na_2SO_4$) and concentrated. Flash chromatography of the crude product (hexane : ethyl acetate : 1:2) gave alcohol compound **4a** as a colourless oil (0.45 g, 96%); $[\alpha]_D^{20}$= +14.3° (c=0.08 in $CHCl_3$); IR (neat), 3650, 3467, 2971, 1679, 1448, 1377, 1288, 1240, 1153, 1137, 971 $cm^{-1}$; $^1$H-NMR (250 MHz, $CDCl_3$); δ in ppm =5.94 (1H, ddd , *J*=15.0, 7.3, 6.4 Hz), 5.82 (1H, dd, *J*= 15.7, 5.3 Hz), 5.59 (2H, m), 5.39 (1H, brs), 5.25 (1H, d, *J*=5.0 Hz), 4.69 (1H, d, *J*=6.6 Hz), 4.54 (1H, d, *J*=6.6 Hz), 4.30 (1H, ddd, *J*=13.2, 6.8, 6.6 Hz), 4.19 (2H, m), 4.02 (2H, m), 3.89 (1H, dd, *J*=7.4, 6.7 Hz), 3.61 (1H, m), 3.35 (3H, s), 2.4-1.8 (5H, m), 1.61 (3H, brs), 1.36 (3H, d, *J*=7.1 Hz), 1.31 (1H, m), 1.19 (3H, d, *J*=6.2 Hz); $^{13}$C-NMR (62.5 MHz, $CDCl_3$), δ in ppm =136.6 (d), 131.6 (d), 131.3 (s), 130.6 (d), 127.1 (d), 120.0 (d), 94.4 (t), 93.6 (d), 80.0 (d), 73.3 (d), 68.4 (d), 67.8 (d), 65.9 (t), 56.0 (d), 37.0 (t), 36.1 (t), 36.0 (t), 23.2 (q), 22.2 (q), 17.5 (q); MS-EI (m/z, %): 620 ($M^+$, 3), 563 (15), 423 (60), 337 (18), 275 (45), 199 (100), 135 (94), HRMS: found: 620,3541 $^+_-$ 5 ppm, $C_{37}H_{52}O_6Si$ requires 620,3533

**xviii) Process for the production of compound 5a**

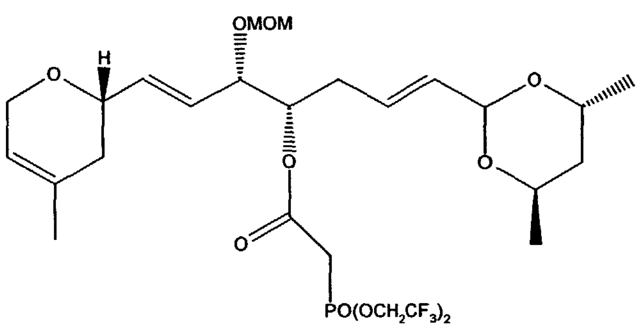

$C_{27}H_{39}F_6O_{10}P$
Exact Mass: 668,2185
Mol. Wt.: 668,5567
C, 48,51; H, 5,88; F, 17,05; O, 23,93; P, 4,63

To a solution of alcohol compound **4a** of step **xvii)** (0,76 g, 2 mmol) and DMAP (0.6 g, 3 mmol) in dry $CH_2Cl_2$ (10 ml) was added slowly a solution of $(CF_3CH_2O)_2P(O)CH_2COCl$ (1.07 g, 1.6 eqv) in $CH_2Cl_2$ at -78°C. After 15 min pyridine (0.3 ml) was added at the same temperature and the mixture was allowed to warm up slowly to room temperature (approximately 2 h). The reaction was quenched with saturated solution of $NaHCO_3$. The organic phase was extracted with $CH_2Cl_2$, (3 X 5 ml). The combined extracts were washed with water followed by brine, dried ($Na_2SO_4$) and concentrated. Flash chromatography of the crude product (hexane: ethyl acetate: 1:1) gave compound **5a** as a colourless oil (0.45 g, 96%); $[\alpha]_D^{20}$= -48° (c=0.25 in $CHCl_3$); IR (neat), 2974, 1740, 1269, 1173, 1104, 964 $cm^{-1}$; $^1H$-NMR (250 MHz, $CDCl_3$); δ in ppm =5.87 (1H, dd *J*=15.7, 5.3 Hz), 5.76 (1H,, t, *J*=6.6 Hz), 5.60 (2H, m), 5.43 (1H, brs), 5.24 (1H, d, *J*=5.0 Hz), 5.07, (1H, ddd *J*=10.0, 8.3, 5.1 Hz), 4.69 (1H, d, *J*=6.9 Hz), 4.54 (1H, d, *J*=6.9 Hz), 4.47 (5H, m), 4.32 (1H, ddd *J*=13.2, 6.8, 1,5 Hz), 4.10 (3H, m), 4.02 (2H, m), 3.37 (3H, s), 3.24 (1H, dd, *J*=16.2, 21.0 Hz), 3.18 (1H, dd, *J*=16.2, 20.4 Hz), 2.5-2.2 (2H, m), 2.1-1.7 (4H, m), 1.71 (3H, brs), 1.38 (3H, d, *J*=7.1 Hz), 1.30 (1H, m), 1.22 (3H, d, *J*=6.8 Hz); $^{13}C$-NMR (62.5 MHz, $CDCl_3$), δ in ppm =160.4 (s), 137. (d), 131.6 (d), 132.1 (d), 131.6 (s), 129.0 (d), 125.8 (d), 120.1 (d), 94.3 (t), 93.3 (d), 76.6 (d), 76.5 (d), 73.3 (d), 68.5 (d), 67.9 (d), 65.9 (t), 63.3 (t), 62.5 (t), 56.1 (d), 37.0 (t), 36.0 (t), 35.4 (t), 33.7 (t), 33.1 (t), 23.2 (q), 22.2 (q), 22.2 (q), 17.5 (q); MS-EI (m/z, %): 668 ($M^+$, 6), 623 (8), 521 (5), 469 (6), 369 (15), 287 (85), 217 (30), 115 (80); HRMS: found: 628,2201$^+$- 5 ppm, $C_{27}H_{39}F_6O_{10}P$ requires 668,2185

**xix) Process for the production of compound 6a**

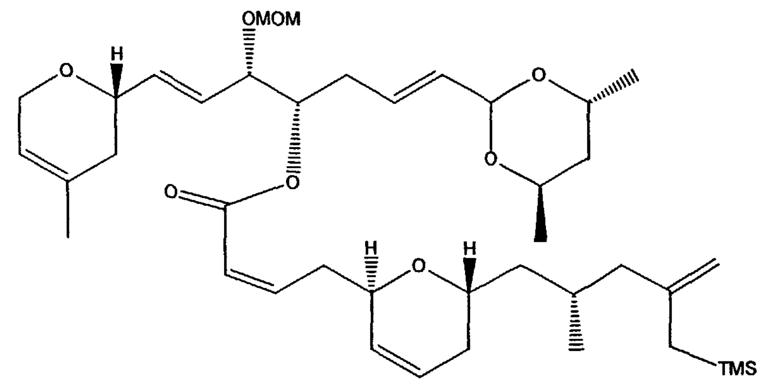

$C_{40}H_{64}O_8Si$
Exact Mass: 700,4370
Mol. Wt.: 701,0169
C, 68,53; H, 9,20; O, 18,26; P, 4,01

To a mixture of compound **5a** of step **xviii)** (0.1 g, 0.15 mmol) and 18-crown-6 (0.21 g, 0,8 mmol) in THF (2 ml) was added KHMDS (0.29 ml, 0.5 M in toluene, 0.15 mmol) at -78°C. After 45 min a solution of aldehyde compound **15** (0.052 g, 0.18 mmol) in THF (1 ml) was added dropwise and the mixture was stirred an additional 45 min. The reaction was quenched with saturated solution of $NH_4Cl$ and extracted with ethyl acetate, (3 X 5 ml). The combined extracts were washed with water followed by brine, dried ($Na_2SO_4$) and concentrated. Flash chromatography of the crude product (hexane : ethyl acetate - 1:1) gave pure (Z)- isomer compound **6a** (0.089g, 85%) as a colourless oil; %); $[\alpha]_D^{20}$= -28° (c=0.2 in $CHCl_3$); IR (neat), 2928, 1719, 1680, 1640, 1560, 1376, 1274, 1164, 1099, 997 $cm^{-1}$; $^1H$-NMR (600 MHz, $CDCl_3$); δ in ppm =6.45 (ddd, *J*=11.5, 7.6, 6.5 Hz),5.91 (1H, dt, *J*=11.5, 1.9 Hz), 5.82 (2H, , m), 5.72 (1H, dq , *J*=9.0, 2.3 Hz), 5.59 (2H, m), 5.40 (1H, brs), 5.24 (1H, d, *J*=5.2 Hz), 5.06 , (1H, ddd, *J*=10.6, 8.5, 5.1 Hz), 4.69 (1H, d, *J*=6.8 Hz), 4.59 (1H, bs), 4.58 (1H, d, *J*=6.8 Hz), 4.56 (1H, brs), 4.32 (1H, ddd *J*=13.4, 6.7, 6.7 Hz), 4.28 (1H, m), 4.19 (3H, m), 4.03 (2H, m), 3.81 (1H, m), 3.37 (3H, s), 3.0. (1H, m),2.93 (1H, m), 2.51 (1H, m), 2.36 (1H, m), 2.1-1.8 (9H, m), 1.71 (3H, brs), 1.67 (2H, m), 1.52 (2H, m), 1.38 (3H, d, *J*=6.9 Hz), 1.35 (1H, brd, *J*=13.3, Hz), 1.23 (3H, d, *J*=6.1 Hz), 1.1 (1H, m), 0.87 (3H, d, *J*=6.4 Hz), 0.3 (9H, s); $^{13}C$-NMR (62.5 MHz, $CDCl_3$), δ in ppm =165.5 (s), 147.9 (d), 146.2 (s), 131.6 (s), 131.3 (d), 129.9 (d), 129.4 (d), 126.4 (d), 125.3 (d), 120.9 (d), 120.1 (d), 109.1 (t), 94.4 (t), 93.5 (d), 76.6 (d), 73.8 (d), 73.5 (d), 72.3 (d), 68.5 (d), 67.8 (d), 65.9 (t), 65.5 (d), 56.1 (d), 47.3 (t), 42.8 (t), 37.1 (t), 36.0 (t), 34.0 (t), 33.6 (t), 31.7 (t), 27.1 (d), 26.7 (t), 23.3 (q), 22.2 (q), 19.7 (q), 17.6 (q); 0.8 (q); MS-EI (m/z, %): 700 ($M^+$, 4), 613 (8), 503 (4), 365 (95), 303 (12), 251 (85),161 (35), 115 (55); HRMS: found 700.4395, requires for $C_{40}H_{64}O_8Si$ 700.4370.

**xx) Process for the production of compound 7a**

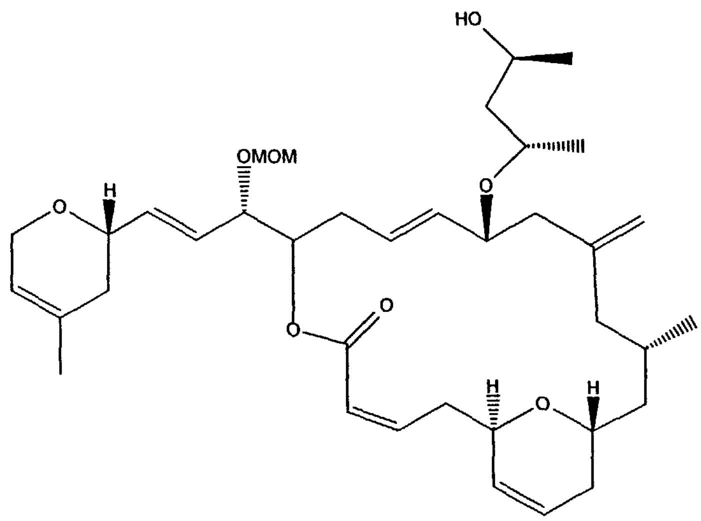

$C_{37}H_{56}O_8$
Exact Mass: 628,3975
Mol. Wt.: 628,8357
C, 70,67; H, 8,98; O, 20,35

To a solution of $EtAlCl_2$ (0.1 ml, 1M in $CH_2Cl_2$, 0.1 mmol) in 15 ml $CH_2Cl_2$ was added slowly (syringe pump), a solution of compound **6a** of step **xix**) (0.04 g,. 0.05 mmol) in $CH_2Cl_2$ (1ml) at -50°C. After the addition was finished, the reaction mixture was allowed to warm up to °C. The reaction was quenched with buffer (pH = 7, 2 ml) and stirred for 2 h. The organic phase phase was separated and the water phase was extracted with $CH_2Cl_2$, (3 X 5 ml). The combined extracts were washed with water followed by brine, dried ($Na_2SO_4$) and concentrated. Flash chromatography of the crude product (hexane: ethyl acetate: 1:1) gave compound **7a** as a colourless oil (0.031 g, 85%); $[\alpha]_D^{20}$= -31° (c=0.1 in $CHCl_3$); IR (neat), 3500, 2928, 17290, 1682, 1638, 1510, 1376, 1164, 990 $cm^{-1}$;6.31 (ddd, *J*=11.5, 10.0, 5.3 Hz), 5.91 (1H, d, *J*=6.9, Hz), 5.84 (2H, , m), 5.71 (1H, dd , *J*=9.9, 1.9 Hz), 5.62.(1H, m), 5.58 (1H, ddd, *J*=7.2, 2.0, 1.3 Hz), 5.47 (dd, *J*=15.5, 8.0 Hz), 5.41 (1H, brs), 5.09, (1H, dd *J*=12.8, 6.4, Hz), 4.79 (1H, brs), 4.76 (1H, brs), 4.67 (1H, d, *J*=6.8 Hz), 4.55 (1H, bs), 4.24 (1H, brs), 4.11(6H, m), 3.96 (1H, ddd *J*=8.3, 8.0, 4.7 Hz), 3.83 (2H, m), 3.62 (1H, m), 3.34 (3H, s), 3.0. (1H, m),2.36 (2H, t, *J*=6.9 Hz),), 2.3-2.0 (5H, m), 1.87 (2H, m), 1.74 (2H, m), 1.60 (2H, m), 1.56 (3H, s), 1.52 (1H, ddd *J*=14.5, 6.3, 2.2 Hz), 1.26 (3H, d, *J*=6.2 Hz), 1.12 (3H, d, *J*=6.1 Hz), 0.85 (3H, d, *J*=6.2 Hz); $^{13}$C-NMR (62.5 MHz, $CDCl_3$), δ in ppm =165.2 (s), 146.9 (d), 144.3 (s), 136.1 (d), 134.2 (d), 131.4 (s), 128.5 (d), 128.4 (d), 126.0 (d), 125.1 (d), 121.7 (d), 119.8 (d), 115.6 (d), 112.9 (t), 94.0 (t), 75.3 (d), 74.0 (d), 73.5 (d), 73.3 (d), 71.9 (d), 69.8 (d), 67.2 (d), 65.9 (t), 64.3 (d), 55.7 (d), 44.8 (d), 44.7 (t), 43.0 (t), 42.8 (t), 38.8 (t), 36.2 (t), 35.9 (t), 34.4 (t), 33.0 (t), 31.) (t), 31.4 (t), 29.8 (t), 28.3 (d), 24.4 (t), 23.5 (q), 23.0 (q), 22.7 (t), 20.0 (q), 18.3 (q), 14.2 (q); HRMS: found: $628.3930^{+}_{-}$ 5 ppm, $C_{37}H_{56}O_8$ requires 628.3975.

## Claims

**1.** Laulimalid derivatives of general formula I

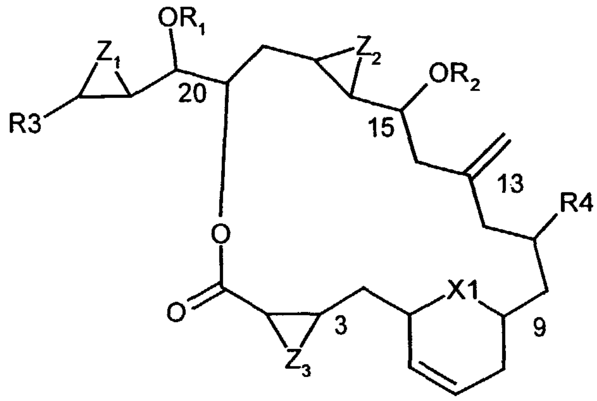

(I),

in which

$R^1$ and $R^2$ independently from each other have the meaning of hydrogen, α-alkyl, β-alkyl, methylmethylether, alkyl optionally substituted with hydroxy, paramethyloxybenzyl, benzyl, or a protecting group,

$R^3$ has the meaning of a five or six membered, optionally substituted aryl or heteroaryl ring, or a five or six membered optionally substituted and optionally partially saturated cycloalkyl ring, which can be interrupted by oxygen, sulphur or the group $=NR^5$,

$R^4$ has the meaning of α-alkyl, β-alkyl, aryl or trifluoromethyl,

$X_1$ has the meaning of oxygen, sulphur or the group $=NR^5$, in which

$R^5$ has the meaning of hydrogen, alkyl, cycloalkyl or aryl,

$Z_1$ has the meaning of a (Z)- or (E)-double bond, triple bond, or has the meaning of oxygen, sulphur or the group $-CH_2-$, $-CH_2-CH_2-$, $=C(OH)_2$, =C(halogen)(OH), $=C(OH)NR^6$ or $=NR^7$,

$Z_2$ has the meaning of a (Z)- or (E)-double bond, triple bond, or has the meaning of oxygen (α- or β-epoxide), sulphur or the group $-CH_2-$, $-CH_2-CH_2$ -, $=C(OH)_2$, =C(halogene)(OH), $=C(OH)NR^6$ or $=NR^7$,

$Z_3$ has the meaning of a (Z) or (E)-double bond,

$R^6$ has the meaning of alkyl, cycloalkyl or aryl, and

$R^7$ has the meaning of hydrogen, alkyl, cycloalkyl or aryl,

and the optical isomers and salts thereof, except of those compounds in which $R^3$ stands for the cycloalkyl ring

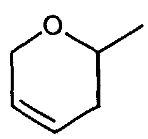

and $X_1$ has the meaning of oxygen, $R^1$ has the meaning of α-methyl, $Z_1$ has the meaning of a (E)-double bond, $Z_2$ has the meaning of β-epoxide if $Z_3$ has the meaning of a (Z)-double bond.

**2.** Laulimalid derivatives of general formula I, according to claim 1, in which

$R^1$ and $R^2$ independently from each other have the meaning of hydrogen, α-$C_1$-$C_6$-alkyl, β- $C_1$-$C_6$-alkyl, methylmethylether, $C_1$-$C_6$-alkyl optionally substituted with hydroxy, paramethyloxybenzyl, benzyl, or a silyl protecting group, or an acetyl, benzyl, carbamate or carbonate protecting group,

$R^3$ has the meaning of a five or six membered, optionally substituted aryl or heteroaryl ring, or a five or six membered optionally substituted and optionally partially saturated $C_3$-$C_7$-cycloalkyl ring, which

can be interrupted by oxygen, sulphur or the group $=NR^5$,

$R^4$ has the meaning of α- $C_1$-$C_6$-alkyl, β- $C_1$-$C_6$-alkyl, aryl or trifluoromethyl,

$X_1$ has the meaning of oxygen, sulphur or the group $=NR^5$, in which

$R^5$ has the meaning of hydrogen, $C_1$-$C_6$-alkyl, $C_3$-$C_7$-cycloalkyl or aryl,

$Z_1$ has the meaning of a (Z)- or (E)-double bond, triple bond, or has the meaning of oxygen, sulphur or the group $-CH_2-$, $-CH_2-CH_2-$, $=C(OH)_2$, =C(halogen)(OH), $=C(OH)NR^6$ or $=NR^7$,

$Z_2$ has the meaning of a (Z)- or (E)-double bond, triple bond, or has the meaning of oxygen (α- or β-epoxide), sulphur or the group $-CH_2-$, $-CH_2-CH_2-$, $=C(OH)_2$, =C(halogene)(OH), $=C(OH)NR^6$ or $=NR^7$,

$Z_3$ has the meaning of a (Z) or (E)-double bond,

$R^6$ has the meaning of $C_1$-$C_6$-alkyl, $C_3$-$C_7$-cycloalkyl or aryl, and

$R^7$ has the meaning of hydrogen, $C_1$-$C_6$-alkyl, $C_3$-$C_7$-cycloalkyl or aryl,

and the optical isomers and salts thereof, except of those compounds in which $R^3$ stands for the cycloalkyl ring

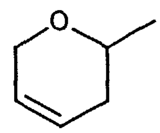

and $X_1$ has the meaning of oxygen, $R^1$ has the meaning of α-methyl, $Z_1$ has the meaning of a (E)-double bond, $Z_2$ has the meaning of β-epoxide if $Z_3$ has the meaning of a (Z)-double bond.

**3.** Laulimalid derivatives of general formula I, according to claims 1 and 2, in which

$R^1$ and $R^2$ independently from each other have the meaning of hydrogen, α-$C_1$-$C_6$-alkyl, β- $C_1$-$C_6$-alkyl, methylmethylether, $C_1$-$C_6$-alkyl optionally substituted with hydroxy, paramethyloxybenzyl, benzyl, or a silyl protecting group, or an acetyl, benzyl, carbamate or carbonate protecting group,

$R^3$ has the meaning of optionally substituted phenyl, biphenyl, naphthyl, thiophene, furan, oxazole, thiazole, imidazole, pyrazole, pyridin, pyrimidine, triazine, quinolin, isoquinolin or benzo derivatives thereof, or a five or six membered optionally substituted and optionally partially saturated $C_3$-$C_7$-cycloalkyl ring, which can be interrupted by oxygen, sulphur or the group $=NR^5$,

$R^4$ has the meaning of α- $C_1$-$C_6$-alkyl, β- $C_1$-$C_6$-alkyl, phenyl, biphenyl, naphthyl, or trifluoromethyl,

$X_1$ has the meaning of oxygen, sulphur or the group $=NR^5$, in which

$R^5$ has the meaning of hydrogen, $C_1$-$C_6$-alkyl, $C_3$-$C_7$-cycloalkyl, phenyl, biphenyl or naphthyl,

$Z_1$ has the meaning of a (Z)- or (E)-double bond, triple bond, or has the meaning of oxygen, sulphur or the group $-CH_2-$, $-CH_2-CH_2-$, $=C(OH)_2$, =C(halogen)(OH), $=C(OH)NR^6$ or $=NR^7$,

$Z_2$ has the meaning of a (Z)- or (E)-double bond, triple bond, or has the meaning of oxygen (α- or β-epoxide), sulphur or the group $-CH_2-$, $-CH_2-CH_2-$, $=C(OH)_2$, =C(halogene)(OH), $=C(OH)NR^6$ or $=NR^7$,

$Z_3$ has the meaning of a (Z) or (E)-double bond,

$R^6$ has the meaning of $C_1$-$C_6$-alkyl, $C_3$-$C_7$-cycloalkyl, phenyl, biphenyl or naphthyl, and

$R^7$ has the meaning of hydrogen, $C_1$-$C_6$-alkyl, $C_3$-$C_7$-cycloalkyl, phenyl, biphenyl or naphthyl,

and the optical isomers and salts thereof, except of those compounds in which $R^3$ stands for the cycloalkyl ring

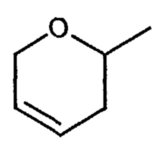

$X_1$ has the meaning of oxygen, $R^1$ has the meaning of α-methyl, $Z_1$ has the meaning of a (E)-double bond, $Z_2$ has the meaning of β-epoxide if $Z_3$ has the meaning of a (Z)-double bond.

**4.** Laulimalid derivatives of general formula I, according to claims 1 to 3, in which

| | |
|---|---|
| $R^1$ and $R^2$ | independently from each other have the meaning of hydrogen, $\alpha$-$C_1$-$C_6$-alkyl, $\beta$- $C_1$-$C_6$-alkyl, methylmethylether, $C_1$-$C_6$-alkyl optionally substituted with hydroxy, paramethyloxybenzyl, benzyl, or a silyl protecting group, or an acetyl, benzyl, carbamate or carbonate protecting group, |
| $R^3$ | has the meaning of optionally substituted phenyl, biphenyl, naphthyl, thiophene, furan, oxazole, thiazole, imidazole, pyrazole, pyridin, pyrimidine, triazine, quinolin, isoquinolin or benzo derivatives thereof, or a five or six membered optionally substituted and optionally partially saturated $C_3$-$C_7$-cycloalkyl ring, which can be interrupted by oxygen, sulphur or the group $=NR^5$, |
| $R^4$ | has the meaning of $\alpha$- $C_1$-$C_6$-alkyl, $\beta$- $C_1$-$C_6$-alkyl, phenyl, biphenyl, naphthyl, or trifluoromethyl, |
| $X_1$ | has the meaning of oxygen, |
| $Z_1$ | has the meaning of a (Z)- or (E)-double bond, or has the meaning of oxygen, |
| $Z_2$ | has the meaning of a (Z)- or (E)-double bond, or has the meaning of oxygen ($\alpha$- or $\beta$-epoxide) and |
| $Z_3$ | has the meaning of a (Z) or (E)-double bond, |

and the optical isomers and salts thereof, except of those compounds in which $R^3$ stands for the cycloalkyl ring

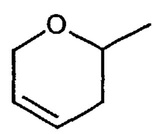

$X_1$ has the meaning of oxygen, $R^1$ has the meaning of $\alpha$-methyl, $Z_1$ has the meaning of a (E)-double bond, $Z_2$ has the meaning of $\beta$-epoxide if $Z_3$ has the meaning of a (Z)-double bond.

**5.** Laulimalid derivatives of general formula I, according to claims 1 to 4, in which

| | |
|---|---|
| $R^1$ and $R^2$ | independently from each other have the meaning of hydrogen, $\alpha$-$C_1$-$C_6$-alkyl, $\beta$- $C_1$-$C_6$-alkyl, methylmethylether, $C_1$-$C_6$-alkyl optionally substituted with hydroxy, paramethyloxybenzyl, benzyl, or a silyl protecting group, or an acetyl, benzyl, carbamate or carbonate protecting group, |
| $R^3$ | has the meaning of optionally substituted phenyl, 1,3-thiazoles or 2- and 3-pyridyl, or a six membered optionally substituted and optionally partially saturated cyclohexyl ring, which can be interrupted by oxygen, |
| $R^4$ | has the meaning of $\alpha$- $C_1$-$C_6$-alkyl, $\beta$- $C_1$-$C_6$-alkyl, phenyl, biphenyl, naphthyl, or trifluoromethyl, |
| $X_1$ | has the meaning of oxygen, |
| $Z_1$ | has the meaning of a (Z)- or (E)-double bond, or has the meaning of oxygen, |
| $Z_2$ | has the meaning of a (Z)- or (E)-double bond, or has the meaning of oxygen ($\alpha$- or $\beta$-epoxide) and |
| $Z_3$ | has the meaning of a (Z) or (E)-double bond, |

and the optical isomers and salts thereof, except of those compounds in which $R^3$ stands for the cycloalkyl ring

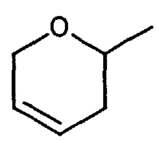

$X_1$ has the meaning of oxygen, $R^1$ has the meaning of $\alpha$-methyl, $Z_1$ has the meaning of a (E)-double bond, $Z_2$ has the meaning of $\beta$-epoxide if $Z_3$ has the meaning of a (Z)-double bond.

**6.** Laulimalid derivatives of general formula I, according to claims 1 to 5, in which

| | |
|---|---|
| $R^1$ and $R^2$ | independently from each other have the meaning of hydrogen, $\alpha$-$C_1$-$C_6$-alkyl, $\beta$- $C_1$-$C_6$-alkyl, methylmethylether, $C_1$-$C_6$-alkyl optionally substituted with hydroxy, paramethyloxybenzyl, benzyl, or a silyl protecting group, or an acetyl, benzyl, carbamate or carbonate protecting group, |
| $R^3$ | has the meaning of optionally substituted phenyl, 1,3-thiazoles, 2- and 3-pyridyl or the group |

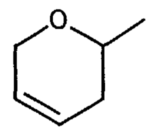

which is substituted with methyl,

$R^4$ has the meaning of α- $C_1$-$C_6$-alkyl, β- $C_1$-$C_6$-alkyl, phenyl, biphenyl, naphthyl, or trifluoromethyl,

$X_1$ has the meaning of oxygen,

$Z_1$ has the meaning of a (Z)- or (E)-double bond,

$Z_2$ has the meaning of a (Z)- or (E)-double bond, and

$Z_3$ has the meaning of a (Z) or (E)-double bond,

and the optical isomers and salts thereof.

**7.** Process for the production of laulimalide and laulimalide derivatives of general formula I

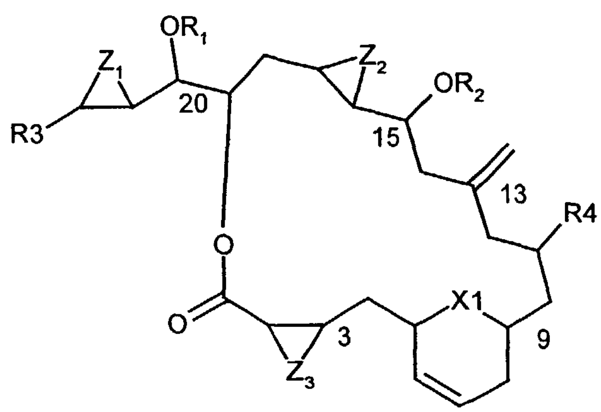

(I),

in which

$R^1$, $R^2$, $R^3$, $R^4$, $X_1$, $Z_1$, $Z_2$ and $Z_3$ have the meaning as defined in claims 1 to 6, **characterised in** by reacting a compound of general formula II

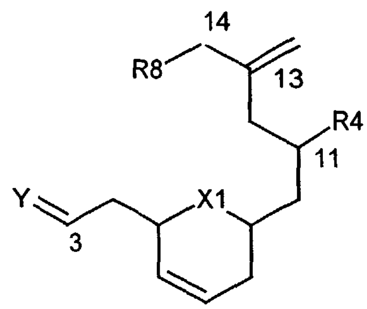

( II ),

in which

| | |
|---|---|
| $R^4$ and $X_1$ | have the meaning defined under formula I, and |
| $R^8$ | has the meaning of hydrogen, trimethylsilyl, or the group MHal, wherein |
| M | has the meaning of Mg, Li, Ti, Ge or In, and |
| Y | has the meaning of oxygen or the group =CH(OH), (Z) or (E)- CH-COOH, (Z) or (E)- =CH-COOR$^9$ or (Z) or (E)- CHHal, in which |
| $R^9$ | has the meaning of hydrogen, alkyl, cycloalkyl or aryl, |

with a compound of general formula III

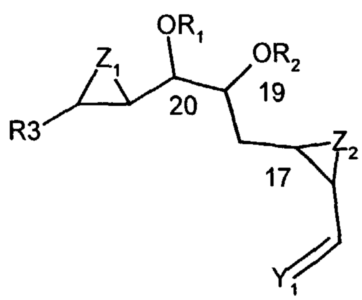

( III ),

in which

| | |
|---|---|
| $R^1$, $R^3$, $Z_1$ und $Z_2$ | have the meaning defined under formula I, and |
| $R^2$ | has the meaning of hydrogen, methylmethylether, paramethyloxybenzyl, benzyl, or a protecting group, or a group $-COCH_2B$, wherein |
| B | has the meaning of the group $-SiR^{10}$, $-SeR^{11}$, $-Se(O)R^{11}$, $-TeR^{11}$, $-PO(OR^{11})_3$ or $-P(O)(OCH_2CR^{10})_2$, in which |
| $R^{10}$ | has the meaning of alkyl, aryl, alkenyl, or the group $-CF_3$, or $-CH_2OR^{11}$, in which |
| $R^{11}$ | has the meaning of hydrogen, alkyl, cycloalkyl or aryl, and |
| $Y_1$ | has the meaning of oxygen, or an alkyl acetal of the group $-CH(OR^{12})_2$, or a five membered O,O; N,O; O,S; or S,S; cyclic acetal, or six membered O,O; N,O; O,S; or S,S; cyclic acetal, and |
| $R^{12}$ | has the meaning of alkyl, |

to form an intermediate of general formula IV

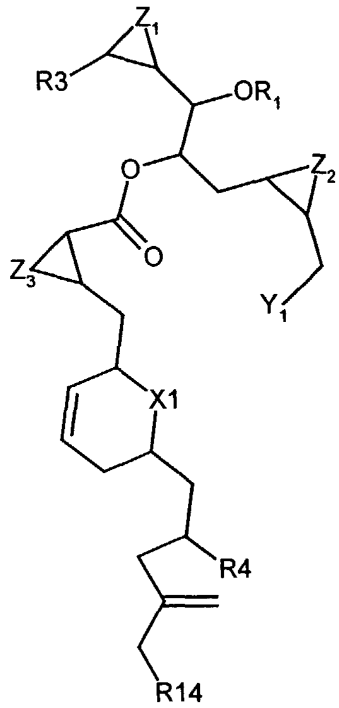

( IV ),

in which

| | |
|---|---|
| $R^1$, $R^3$, $R^4$, $X_1$ and $Z_1$-$Z_3$ | have the meaning defined under formula I, and |
| $Y_1$ | has the meaning defined under formula III, and |
| $R^{14}$ | has the meaning of hydrogen, halogen, trimethylsilyl, or the group MHal, wherein |
| M | has the meaning of Mg, Li, Ti, Ge or In, |

which is cyclidized to the compounds of general formula I.

**8.** Process for the production of intermediates of general formula II,

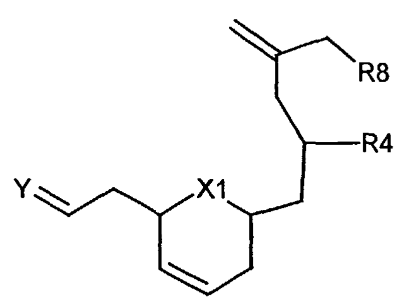

II),

in which $R^4$ and $X_1$ have the meaning as defined under formula I, and $R^8$ has the meaning of hydrogen, trimethylsilyl, or the group MHal, wherein M has the meaning of Mg, Li, Ti, Ge or In, and Y has the meaning of oxygen or the group =CH(OH), (Z) or (E)-CH-COOH, (Z) or (E)- =CH-COOR$^9$ or (Z) or (E)-CHHal, in which $R^9$ has the meaning of hydrogen, alkyl, cycloalkyl or aryl, **characterized in** by reacting

a) an alcohol compound of general formula II-2)

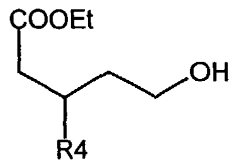

II-2),

in which $R^4$ has the meaning as defined under formula I, in a suitable solvent together with a suitable oxidant to give an aldehyde compound of general formula II-3)

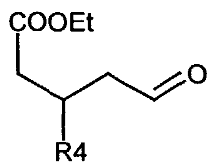

II-3),

in which $R^4$ has the meaning as defined under formula I,

b) reacting aldehyde compound of general formula II-3) in a suitable solvent with a suitable alkylating agent to give a compound of general formula II-4)

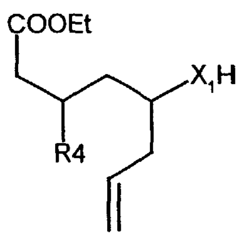

II-4),

in which $R^4$ and $X_1$ have the meaning as defined under formula I,

c) reacting compound II-4) with an amine of general formula II-4a

$$H - NR^iR^{ii} \qquad (II\text{-}4a),$$

in which $R^i$ and $R^{ii}$ independently from each other have the meaning of hydrogen or $C_1$-$C_6$-alkyl, to give a compound of general formula II-5)

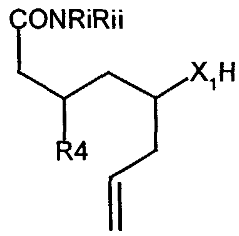

II-5),

in which $R^4$ and $X_1$ have the meaning as defined under formula I and $R^i$ and $R^{ii}$ have the meaning as defined under formula II-4a,

d) reacting compound II-5) in a suitable solvent with a suitable acetal and a suitable hydroxy compound to give a compound of general formula II-6)

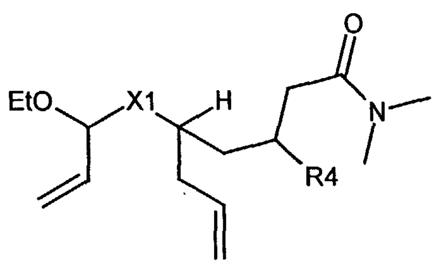

II-6),

in which $R^4$ and $X_1$ have the meaning as defined under formula I,

e) deoxygenating compound II-6) in a suitable solvent with a suitable catalyst, to give a compound of general formula II-7)

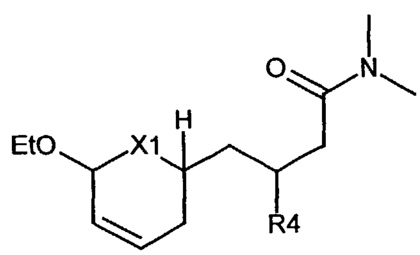

II-7),

in which $R^4$ and $X_1$ have the meaning as defined under formula I,

f) reacting compound II-7) with a suitable vinyl ether and a suitable perchlorate in a suitable solvent, to give a compound of general formula II-8)

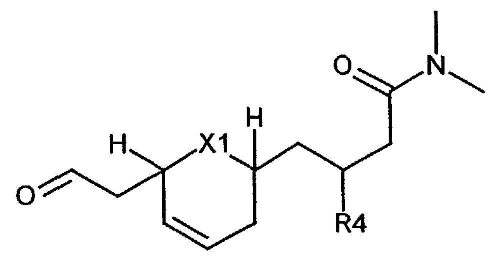

II-8)

in which $R^4$ and $X_1$ have the meaning as defined under formula I,

g) reacting aldehyde compound II-8) with a suitable reductant in a suitable solvent to give an alcohol compound of general formula II-9)

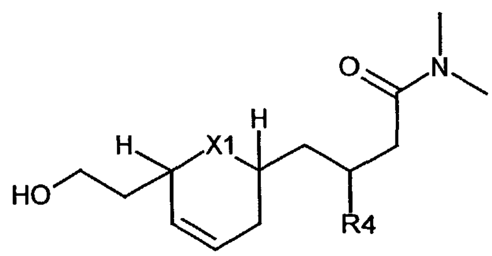

II-9)

in which $R^4$ and $X_1$ have the meaning as defined under formula I,

h) reacting a solution of alcohol compound II-9) in a suitable organic base and with a silyl chloride or a triflak (Si) of general formula II-9a)

$$R^{21}R^{22}R^{23}\text{-SiCl or } R^{21}R^{22}R^{23}\text{SiOTf} \qquad \text{(II-9a)},$$

in which $R^{21}$, $R^{22}$ and $R^{23}$ independently from each other have the meaning of $\alpha$- or $\beta$-$C_1$-$C_6$-alkyl, to give an amide compound of general formula II-10)

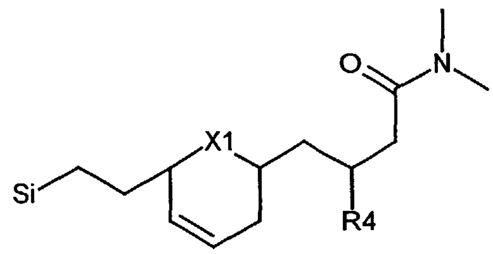

II-10)

in which $R^4$ and $X_1$ have the meaning as defined under formula I and Si has the meaning as defined under formula II-9a,

i) reacting amide compound of general formula II-10) with a metal organic compound and in a suitable solvent to give a ketone compound of general formula II-11)

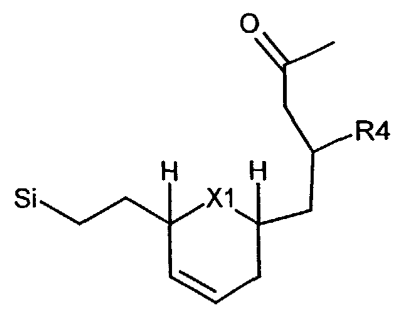

II-11),

in which $R^4$ and $X_1$ have the meaning as defined under formula I and Si has the meaning as defined under formula II-9a,

j) reacting the ketone compound II-11) with a suitable strong amide base in a suitable solvent, and accordingly with a suitable acylating agent in a suitable solvent to give an enoltriflate of general formula II-12)

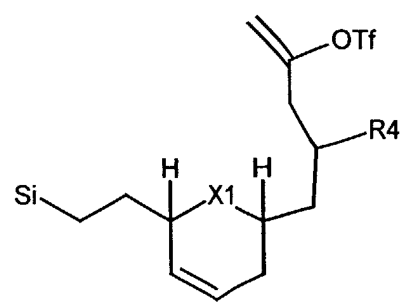

II-12)

in which $R^4$ and $X_1$ have the meaning as defined under formula I and Si has the meaning as defined under formula II-9a,

k) reacting enoltriflate compound II-12) with a suitable Pd catalyst and with alkali chloride in a suitable solvent, and accordingly with $TMSCH_2MgCl$ to give compound II-13)

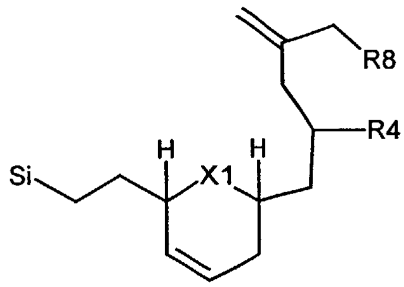

II-13)

in which $R^4$ and $X_1$ have the meaning as defined under formula I, $R^8$ has the meaning as defined under formula II, and Si has the meaning as defined under formula II-9a,

l) reacting compounds of general formula II-13) with a suitable ionic fluoride in a suitable solvent to give compound II-14)

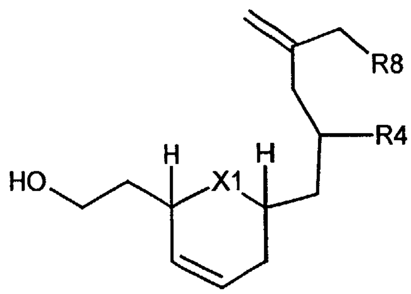

II-14)

in which $R^4$ and $X_1$ have the meaning as defined under formula I and $R^8$ has the meaning as defined under formula II,

m) reacting compound II-14) with a suitable oxidant in a suitable solvent to give an aldehyde compound of general formula II-15)

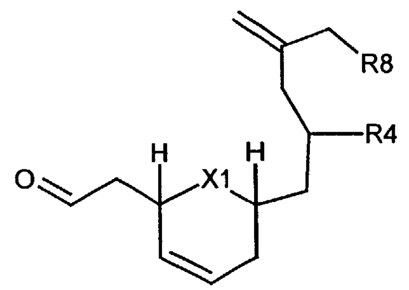

II-15)

in which $R^4$ and $X_1$ have the meaning as defined under formula I and $R^8$ has the meaning as defined under formula II, and

n) reacting aldehyde compound II-15) with a phosphonyl acetic ester of general formula II-15a)

$$(R^xO)_2P(O)CH_2CO_2R^y \quad \text{(II-15a)},$$

in which $R^x$ and $R^y$ independently have from each other have the meaning of $C_1$-$C_6$-alkyl, halo-$C_1$-$C_6$-alkyl or phenyl, and with 18-crown-6, and a suitable strong amid base, in a suitable solvent to give the intermediate compound of general fomula II).

**9.** Process for the production of general formula III,

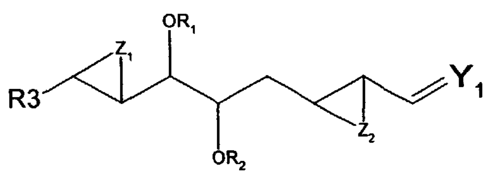

( III ),

in which

| | |
|---|---|
| $R^1$, $R^3$, $Z_1$ and $Z_2$ | have the meaning defined under formula I, and |
| $R^2$ | has the meaning of hydrogene, methylmethylether, paramethyloxybenzyl, benzyl, or a protecting group, or a group $-COCH_2B$, wherein |
| B | has the meaning of the group $-SiR^{10}$, $-SeR^{11}$, $-Se(O)R^{11}$, $-TeR^{11}$, $-PO(OR^{11})_3$ or $-P(O)(OCH_2CR^{10})_2$, in which |
| $R^{10}$ | has the meaning of alkyl, aryl, alkenyl, or the group $-CF_3$, or $-CH_2OR^{11}$, in which |
| $R^{11}$ | has the meaning of hydrogene, alkyl, cycloalkyl or aryl, and |
| $Y_1$ | has the meaning of oxygene, or an alkyl acetal of the group $-CH(OR^{12})_2$, or a five membered O,O; N,O; O,S; or S,S; cyclic acetal, or six membered O,O; N,O; O,S; or S,S; cyclic acetal, and |
| $R^{12}$ | has the meaning of alkyl, which is **characterized in** by reacting |

o) an alcohol compound of general Formula III-16)

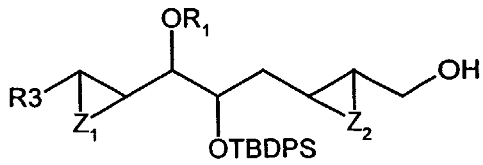

III-16),

in which $R^1$, $R^3$, $Z_1$ and $Z_2$ have the meaning as defined under general formula I, with a suitable oxidant in a suitable solvent to give an aldehyde compound of general formula III-17)

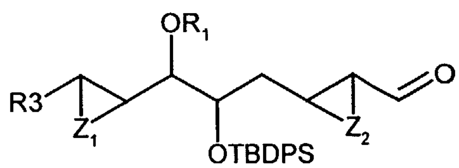

III-17),

in which $R^1$, $R^3$, $Z_1$ and $Z_2$ have the meaning as defined under general formula I,

p) reacting a compound of general formula III-17) with an acetal in a suitable solvent to give a compound of general Formula III-18)

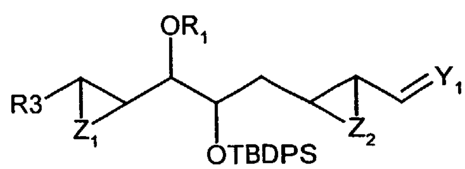

III-18),

in which $R^1$, $R^3$, $Z_1$ and $Z_2$ have the meaning as defined under general formula I, and $Y_1$, has the meaning as defined under formula III,

q) reacting a compound of general formula III-18) with a suitable ionic fluoride in a suitable solvent to an alcohol compound of general formula III-19)

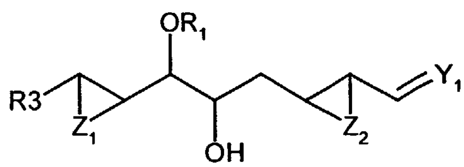

III-19),

in which $R^1$, $R^3$, $Z_1$ and $Z_2$ have the meaning as defined under general formula I, and $Y_1$ has the meaning as defined under formula III, and

r) reacting a compound of general formula III-19) with a suitable tertiary amine in a suitable solvent, and accordingly with a solution of a phosphoro acetyl chloride of general formula III-19a

$$(R^qO)_2P(O)CH_2COCl \qquad \text{(III-19a)},$$

in which $R^q$ has the meaning of $C_1$-$C_6$-alkyl, halo-$C_1$-$C_6$-alkyl or aryl, in a suitable organic base to give a compound of general formula III.

**10.** Compounds of general formula II

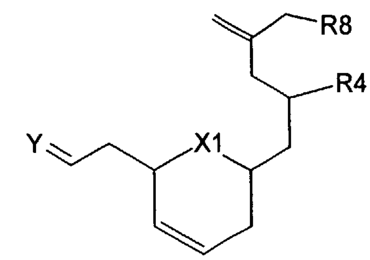

( II ),

in which

| | |
|---|---|
| $R^4$ and $X_1$ | have the meaning defined above under formula I, and |
| $R^8$ | has the meaning of hydrogen, trimethylsilyl, or the group MHal, wherein |
| M | has the meaning of Mg, Li, Ti, Ge or In, and |
| Y | has the meaning of oxygen or the group =CH(OH), (Z) or (E)- CH-COOH, (Z) or (E)- =CH-COOR$^9$ or (Z) or (E)-CHHal, in which |
| $R^9$ | has the meaning of hydrogen, alkyl, cycloalkyl or aryl, as intermediates for the production of compounds of general formula I. |

**11.** Compounds of general formula III

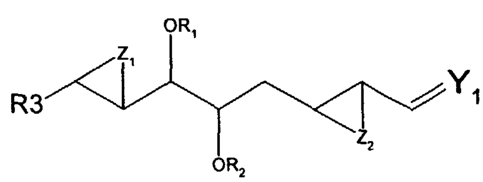

( III ),

in which

| | |
|---|---|
| $R^1$, $R^3$, $Z_1$ und $Z_2$ | have the meaning defined above under formula I, and |
| $R^2$ | has the meaning of hydrogene, methylmethylether, paramethyloxybenzyl, benzyl, or a protecting group, or a group -$COCH_2B$, wherein |

| | |
|---|---|
| B | has the meaning of the group $-SiR^{10}$, $-SeR^{11}$, $-Se(O)R^{11}$, $-TeR^{11}$, $-PO(OR^{11})_3$ or $-P(O)(OCH_2CR^{10})_2$, in which |
| $R^{10}$ | has the meaning of alkyl, aryl, alkenyl, or the group $-CF_3$, or $-CH_2OR^{11}$, in which |
| $R^{11}$ | has the meaning of hydrogene, alkyl, cycloalkyl or aryl, and |
| $Y_1$ | has the meaning of oxygene, or an alkyl acetal of the group $-CH(OR^{12})_2$, or a five membered O,O; N,O; O,S; or S,S; cyclic acetal, or six membered O,O; N,O; O,S; or S,S; cyclic acetal, and |
| $R^{12}$ | has the meaning of alkyl, as intermediates for the production of compounds of general formula I. |

**12.** Compounds of general formula IV

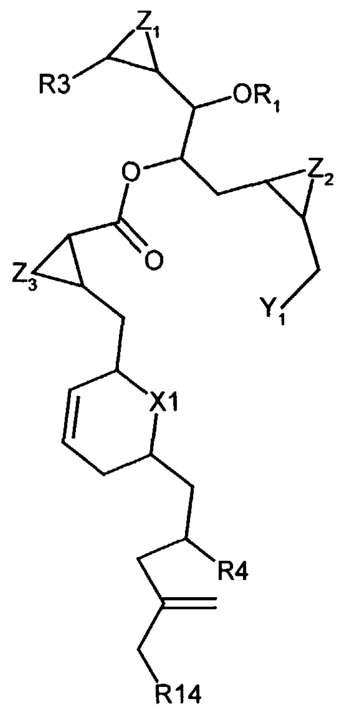

( IV ),

in which

| | |
|---|---|
| $R^1$, $R^3$, $R^4$, $X_1$ and $Z_1$-$Z_3$ | have the meaning defined above under formula I, and |
| $Y_1$ | has the meaning defined above under formula III, and |
| $R^{14}$ | has the meaning of hydrogen, halogen, trimethylsilyl, or the group MHal, wherein |
| M | has the meaning of Mg, Li, Ti, Ge or In, as intermediates for the production of compounds of general formula I. |

**13.** Use of compounds of general formula I, according to claims 1 to 6, for the treatment of cancer, such as solide tumors and Leukemia, autoimmune diseases, such as psoriasis, alopezia and multiple sklerose, chemotherapeutically induced alopezia and mukositis, cardiovascular diseases, such as stenosis, arteriosclerosis and restenosis, infectious diseases caused by unicellulare parasites, such as trypanosoma, toxoplasma or plasmodium, or nephrological diseases caused by fungi, such as glomerulonephritis, chronical neurodegenerative diseases, such as Huntington's disease, amyotropical lateral sclerose, Parkinson disease, AIDS dementia and Alzheimer's diseases, acute neurodegenerative disease, such as ichemia of the brain and neurotraumata, virale infektions, such as wie

z. B. Cytomegalus-infectiones, herpes, hepatitis B and C, and HIV diseases.

**14.** A medicament comprising one or more compounds of general Formula I, according to claim1 to 6.

**15.** A medicament according to claim 14, together with suitable formulations and vehicles.

European Patent Office

# EUROPEAN SEARCH REPORT

Application Number
EP 01 25 0331

| Category | Citation of document with indication, where appropriate, of relevant passages | Relevant to claim | CLASSIFICATION OF THE APPLICATION (Int.Cl.7) |
|---|---|---|---|
| X | GHOSH, A. K. ET AL: "A macrolactonization-based strategy to obtain microtubule- stabilizing agent (-)-laulimalide" TETRAHEDRON LETT. (2001), 42(20), 3399-3401 , XP002189881 * compounds 1, 8,9,15 * | 1 | C07D493/08 C07D493/18 C07F7/08 C07D309/26 C07F9/655 C07D407/14 //(C07D493/08, 321:00, 311:00), (C07D493/18, 321:00,311:00, 311:00) |
| X | GHOSH, ARUN K. ET AL: "Total Synthesis of (-)-Laulimalide" JOURNAL OF THE AMERICAN CHEMICAL SOCIETY (2000), 122(44), 11027-11028 , XP002189882 * compounds 1, 19, 20 * | 1 | |
| X | WO 01 54689 A (UNIV HAWAII ;UNIV UTAH STATE (US)) 2 August 2001 (2001-08-02) * claim 7, compounds 1-4,6 * | 1 | |
| | | | TECHNICAL FIELDS SEARCHED (Int.Cl.7) |
| | | | C07D C07F |

The present search report has been drawn up for all claims

| Place of search | Date of completion of the search | Examiner |
|---|---|---|
| THE HAGUE | 11 February 2002 | Alfaro Faus, I |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT ON EUROPEAN PATENT APPLICATION NO.** EP 01 25 0331

This annex lists the patent family members relating to the patent documents cited in the above-mentioned European search report.
The members are as contained in the European Patent Office EDP file on
The European Patent Office is in no way liable for these particulars which are merely given for the purpose of information.

11-02-2002

| Patent document cited in search report | Publication date | Patent family member(s) | Publication date |
|---|---|---|---|
| WO 0154689 A | 02-08-2001 | AU 3117301 A | 07-08-2001 |
| | | WO 0154689 A1 | 02-08-2001 |

EPO FORM P0459

For more details about this annex : see Official Journal of the European Patent Office, No. 12/82